# EUROPEAN PATENT SPECIFICATION

(11) **EP 4 386 076 B1**
(45) Date of publication and mention of the grant of the patent: **24.06.2026**
(21) Application number: 22855905.0
(22) Date of filing: 10.08.2022
(51) Int. Cl.: C12M 1/00, C12Q 1/02, G16H 30/00, G16H 50/20, G01N 33/48, G06V 20/69

(54) **DIAGNOSIS DEVICE, INFORMATION ACQUISITION METHOD, AND PROGRAM**
DIAGNOSEVORRICHTUNG, INFORMATIONSERFASSUNGSVERFAHREN UND PROGRAMM
DISPOSITIF DE DIAGNOSTIC, PROCÉDÉ D'ACQUISITION D'INFORMATIONS ET PROGRAMME

(30) Priority: 12.08.2021 JP 2021131711
(43) Date of publication of application: 19.06.2024
(73) Proprietor: KURUME UNIVERSITY, Kurume-shi Fukuoka 830-0011 (JP)
(72) Inventor: OHSHIMA Koichi, Kurume-shi, Fukuoka 830-0011 (JP); MIYOSHI Hiroaki, Kurume-shi, Fukuoka 830-0011 (JP); SATO Kensaku, Kurume-shi, Fukuoka 830-0011 (JP); NAGAISHI Miharu, Kurume-shi, Fukuoka 830-0011 (JP); TAKEUCHI Ichiro, Nagoya-shi, Aichi 466-8555 (JP); HONTANI Hidekata, Nagoya-shi, Aichi 466-8555 (JP)
(74) Representative: v. Bezold & Partner Patentanwälte - PartG mbB
(86) International application number: PCT/JP2022/030614
(87) International publication number: WO 2023/017843

(56) References cited:
- DE-A1- 102015 114 026
- JP-A- 2001 059 842
- JP-A- 2004 286 666
- JP-A- 2020 180 954
- US-A1- 2012 082 365
- RYOICHI KOGA ET AL: "Extracting and Visualization of Essential Features for Staining Translation of Pathological Images", vol. 119, no. 399 (MI2019-116), 30 November 2019 (2019-11-30), pages 215 - 218, XP009543612, Retrieved from the Internet <URL:https://ken.ieice.org/ken/paper/20200130I1uO/eng/>
- KOGA RYOICHI ET AL: "Detection of DLBCL regions in H&E stained whole slide pathology images using Bayesian U-Net", PROCEEDINGS OF THE SPIE, SPIE, US, vol. 11792, 20 April 2021 (2021-04-20), pages 1179203 - 1179203, XP060140803, ISSN: 0277-786X, ISBN: 978-1-5106-5738-0, DOI: 10.1117/12.2590719
- KOGA RYOICHI ET AL: "Stain transfer for automatic annotation of malignant lymphoma regions in H&E stained whole slide histopathology images", PROCEEDINGS OF THE SPIE, SPIE, US, vol. 11792, 20 April 2021 (2021-04-20), pages 117920R - 117920R, XP060140812, ISSN: 0277-786X, ISBN: 978-1-5106-5738-0, DOI: 10.1117/12.2590720
- RYOICHI KOGA, NORIAKI HASHIMOTO, TATSUYA YOKOTA (NIT), MASATO NAKAGURO, KEI KOHNO, SHIGEO NAKAMURA (NUI), ICHIRO TAKEUCHI, HIDEKAT: "Extracting and Visualization of Essential Features for Staining Translation of Pathological Images", IEICE TECHNICAL REPORT; MI, IEICE, JP, vol. 119, no. 399 (MI2019-116), 30 November 2019 (2019-11-30), JP, pages 215 - 218, XP009543612

## Description

### Technical Field

The present disclosure relates to a diagnosis device, an information acquisition method, and a program.

### Background Art

Malignant lymphomas are the most frequent blood tumors in adults in Japan. Early stage malignant lymphomas hardly causing subjective symptoms are diseases that are difficult to early recognize (see Patent Literature 1). Malignant lymphomas can be developed in any organ throughout the body, and are classified to more than 100 subtypes of disease, including not only those frequently developed, but also those very rarely developed. Malignant lymphomas are considerably different in therapeutic strategy with respect to each subtype of disease, and thus identification of the subtype of disease based on pathological diagnoses has a great influence on a patient's prognosis. Furthermore, Patent Literature 2 discloses a pathologic tissue image analyzing system which supports pathologic tissue image diagnoses performed by analyzing biological tissue images.

### Citation List

### Patent Literature

Patent Literature 1: Unexamined Japanese Patent Application Kokai Publication No. 2016-80672
Patent Literature 2: US 2012/082365 A1

### Summary of Invention

### Technical Problem

Malignant lymphomas are definitively diagnosed after pathological diagnoses mainly by experienced pathologist with skilled knowledge and experience after sharing of the results together with physicians. There has not been constructed any method of diagnosis from pathological tissues based on an objective index and thus it is difficult to accurately share information from pathological tissues, between physicians, and an example has also been caused where no unified discretion can be obtained from physicians making diagnoses (different diagnoses are made). While various novel therapeutic methods suitable for each subtype of disease have been developed, there is a demand for development of a diagnosis device in which a unified diagnostic index for diagnosing malignant lymphoma can be provided, in order to accurately identify various subtypes of disease and then lead to an appropriate therapy.

The present disclosure has been made in view of the above circumstances, and an objective thereof is to provide a diagnosis device, an information acquisition method, and a program, in which a unified diagnostic index for diagnosing malignant lymphoma can be provided.

### Solution to Problem

In order to achieve the above objective, a diagnosis device according to a first aspect of the present disclosure includes
an extractor that extracts an individual cell nucleus, an individual cell, and a distribution state of cell from a pathological image in which a plurality of cells including a lymphoid cell is photographed, and
a digitizer that digitizes a feature of an individual cell nucleus, a feature of an individual cell, and a feature of a distribution state of cell based on the individual cell nucleus, the individual cell, and the distribution state of cell extracted by the extractor.

In this case, the extractor may
read a pathological image obtained by taking a range in which a feature of a distribution state of cell is recognizable, at a resolution at which features of an individual cell nucleus and an individual cell are recognizable, and
extract an individual cell nucleus, an individual cell, and a distribution state of cell from the pathological image read.

The extractor may
perform machine learning based on sample data representing a relationship between a sample image as a sample of the pathological image, and an individual cell nucleus, an individual cell and a distribution state of cell manually extracted from the sample image, and
extract an individual cell nucleus, an individual cell, and a distribution state of cell from the pathological image, based on a result of the machine learning.

The extractor may
not only extract an individual cell, an individual cell nucleus and a distribution state of cell from a first image having a resolution at which an individual cell nucleus and an individual cell taken from a living organism are recognizable, but also extract a distribution state of cell from a second image containing a range in which a feature of a distribution state of cell taken from the living organism is recognizable.

The extractor may
not only perform machine learning based on sample data representing a relationship between a first sample image corresponding to the first image, and a cell and a cell nucleus manually extracted from the first sample image, but also perform machine learning based on sample data representing a relationship between a second sample image corresponding to the second image, and a distribution state of cell manually extracted from the second sample image, and
not only extract an individual cell and an individual cell nucleus from the first image, but also extract a distribution state of cell from the second image, based on a result of the machine learning.

The digitizer may
digitize a feature of an individual cell nucleus, a feature of an individual cell and a feature of a distribution state of cell correlated with information on malignant lymphoma, based on the individual cell nucleus, the individual cell, and the distribution state of cell extracted by the extractor.

The digitizer may
perform machine learning based on sample data representing a relationship between a feature of an individual cell nucleus, a feature of an individual cell and a feature of a distribution state of cell, and information on malignant lymphoma, and
narrow a feature of an individual cell nucleus, a feature of an individual cell and a feature of a distribution state of cell, down to a feature to be digitized, based on a result of the machine learning.

An estimator is included which estimates information on malignant lymphoma based on a feature of an individual cell nucleus, a feature of an individual cell and a feature of a distribution state of cell, digitized by the digitizer.

The estimator
performs machine learning based on sample data representing a relationship between a feature of an individual cell nucleus, a feature of an individual cell and a feature of a distribution state of cell, and information on malignant lymphoma, and
estimates information on malignant lymphoma from a feature of an individual cell nucleus, a feature of an individual cell and a feature of a distribution state of cell, digitized by the digitizer, based on a result of the machine learning.

The estimator
estimates information on malignant lymphoma based on not only an individual cell nucleus, a feature of an individual cell and a feature of a distribution state of cell, digitized by the digitizer, but also at least one additional information of genome information, gene expression information, protein information or clinical information correlated with malignant lymphoma.

Information on malignant lymphoma may include at least one of information representing presence/absence, a class or a progress stage of malignant lymphoma, or genome information, gene expression information, protein information or clinical information correlated with malignant lymphoma.

The estimator may
perform machine learning based on sample data representing a relationship between a feature of an individual cell nucleus, a feature of an individual cell, a feature of a distribution state of cell and the additional information, and information on malignant lymphoma, and
estimate information on malignant lymphoma based on a result of the machine learning.

The cell may include a vascular endothelial cell, a mesenchymal cell including a fibroblast cell, a blood cell other than a lymphoid cell, including histiocyte, an epithelial cell, or a neural cell.

An information acquisition method according to a second aspect of the present disclosure is
an information acquisition method executed by an information-processing device, wherein the processing is to be executed by the diagnosis device according to the present invention as claimed, the method including
extracting an individual cell nucleus, an individual cell, and a distribution state of cell from a pathological image in which a plurality of cells including a lymphoid cell is photographed, and
digitizing a feature of an individual cell nucleus, a feature of an individual cell, and a feature of a distribution state of cell based on the extracted individual cell nucleus, the extracted individual cell, and the extracted distribution state of cell.

A program according to a third aspect of the present disclosure causes
a computer to serve as a diagnosis device according to the present invention as claimed.

### Advantageous Effects of Invention

According to the present disclosure, features of an individual cell nucleus, an individual cell and a distribution state of cell in a plurality of cells including a lymphoid cell, variously changed depending on suffering from malignant lymphoma, are digitized. An individual cell nucleus, an individual cell and a distribution state of cell are changed depending on suffering from malignant lymphoma, and are different in feature of change with respect to each class. Therefore, features digitized, of an individual cell nucleus, an individual cell and a distribution state of individual cell, serve as an objective and unified index in a diagnosis of malignant lymphoma. Accordingly, the present disclosure can provide a unified diagnostic index for diagnosing malignant lymphoma.

### Brief Description of Drawings

FIG. 1 is a block diagram illustrating a configuration of a diagnosis device according to Embodiment 1 of the present disclosure;
FIG. 2A illustrates one example of a pathological image in which a cell nucleus for input in the diagnosis device in FIG. 1 can be confirmed;
FIG. 2B illustrates one example of a pathological image in which a distribution state for input in the diagnosis device in FIG. 1 can be confirmed;
FIG. 2C illustrates one example of a dividable pathological image;
FIG. 3 is a block diagram illustrating a configuration of sample data used in machine learning in an extractor constituting the diagnosis device in FIG. 1;
FIG. 4 is a block diagram illustrating a configuration of sample data used in machine learning in a digitizer constituting the diagnosis device in FIG. 1;
FIG. 5 is a block diagram illustrating a hardware configuration of the diagnosis device in FIG. 1;
FIG. 6 is a flowchart illustrating a flow of learning stage processing in the diagnosis device in FIG. 1;
FIG. 7A is a block diagram illustrating a flow of machine learning in an extractor constituting the diagnosis device in FIG. 1;
FIG. 7B is a first example of a graph representing characteristics of numerical information on a feature of a cell used in machine learning in a digitizer constituting the diagnosis device in FIG. 1;
FIG. 7C is a second example of a graph representing characteristics of numerical information on a feature of a cell used in machine learning in a digitizer constituting the diagnosis device in FIG. 1;
FIG. 8 is a flowchart illustrating a flow of diagnosis stage processing in the diagnosis device in FIG. 1;
FIG. 9 is a block diagram illustrating a configuration of a diagnosis device according to Embodiment 2 of the present disclosure;
FIG. 10 is a block diagram illustrating a configuration of sample data used in machine learning in an estimator constituting the diagnosis device in FIG. 9;
FIG. 11 is a flowchart illustrating a flow of learning stage processing in the diagnosis device in FIG. 9;
FIG. 12 is a block diagram illustrating machine learning in an estimator constituting the diagnosis device in FIG. 9;
FIG. 13 is a flowchart illustrating a flow of diagnosis stage processing in the diagnosis device in FIG. 9;
FIG. 14 is a block diagram illustrating a configuration of a diagnosis device according to Embodiment 3 of the present disclosure;
FIG. 15 is a block diagram illustrating a variant of a configuration of the diagnosis device in FIG. 14;
FIG. 16 is a block diagram illustrating a configuration of a diagnosis device according to Embodiment 4 of the present disclosure;
FIG. 17 is a block diagram illustrating a configuration of sample data used in machine learning in an extractor constituting the diagnosis device in FIG. 16;
FIG. 18A is a graph representing a first axis coefficient in a two-dimensional scatter diagram obtained by linear discriminant analysis of a cell nucleus form performed with parameters representing 22 features;
FIG. 18B is a graph representing a second axis coefficient in a two-dimensional scatter diagram obtained by linear discriminant analysis of a cell nucleus form performed with parameters representing 22 features;
FIG. 19A is a graph representing distribution states of features of a cell nucleus of DLBCL and a cell nucleus of FL in a first axis direction, in the case of linear discriminant analysis performed with parameters representing a feature of a cell nucleus form;
FIG. 19B is a graph representing the first axis coefficient values in the case of FIG. 19A;
FIG. 19C is a graph representing distribution states of features of a cell nucleus of DLBCL and a cell nucleus of Reactive in a first axis direction, in the case of linear discriminant analysis performed with parameters representing a feature of a cell nucleus form;
FIG. 19D is a graph representing the first axis coefficient values in the case of FIG. 19C;
FIG. 19E is a graph representing distribution states of features of a cell nucleus of FL and a cell nucleus of Reactive in a first axis direction, in the case of linear discriminant analysis performed with parameters representing a feature of a cell nucleus form;
FIG. 19F is a graph representing the first axis coefficient values in the case of FIG. 19E;
FIG. 20A is a graph representing a first axis coefficient in a two-dimensional scatter diagram obtained by linear discriminant analysis performed with parameters representing a feature about the shape (internal texture) of a cell nucleus;
FIG. 20B is a graph representing a second axis coefficient in a two-dimensional scatter diagram obtained by linear discriminant analysis performed with parameters representing a feature about the shape (internal texture) of a cell nucleus;
FIG. 21A is a graph representing distribution states of features of a cell nucleus of DLBCL and a cell nucleus of FL in a first axis direction, in the case of linear discriminant analysis performed with parameters representing a feature about a shape;
FIG. 21B is a graph representing the first axis coefficient values in the case of FIG. 21A;
FIG. 21C is a graph representing distribution states of features of a cell nucleus of DLBCL and a cell nucleus of Reactive in a first axis direction, in the case of linear discriminant analysis performed with parameters representing a feature about a shape;
FIG. 21D is a graph representing the first axis coefficient values in the case of FIG. 21C;
FIG. 21E is a graph representing distribution states of features of a cell nucleus of FL and a cell nucleus of Reactive in a first axis direction, in the case of linear discriminant analysis performed with parameters representing a feature about a shape;
FIG. 21F is a graph representing the first axis coefficient values in the case of FIG. 21E;
FIG. 22A is a graph representing a first axis coefficient in a two-dimensional scatter diagram obtained by linear discriminant analysis of a cell nucleus form performed with parameters representing a feature about the color of a cell nucleus;
FIG. 22B is a graph representing a second axis coefficient in a two-dimensional scatter diagram obtained by linear discriminant analysis of a cell nucleus form performed with parameters representing a feature about the color of a cell nucleus;
FIG. 23A is a graph representing distribution states of features of a cell nucleus of DLBCL and a cell nucleus of FL in a first axis direction, in the case of linear discriminant analysis performed with parameters representing a feature about a color;
FIG. 23B is a graph representing the first axis coefficient values in the case of FIG. 23A;
FIG. 23C is a graph representing distribution states of features of a cell nucleus of DLBCL and a cell nucleus of Reactive in a first axis direction, in the case of linear discriminant analysis performed with parameters representing a feature about a color;
FIG. 23D is a graph representing the first axis coefficient values in the case of FIG. 23C;
FIG. 23E is a graph representing distribution states of features of a cell nucleus of FL and a cell nucleus of Reactive in a first axis direction, in the case of linear discriminant analysis performed with parameters representing a feature about a color;
FIG. 23F is a graph representing the first axis coefficient values in the case of FIG. 23E;
FIG. 24A is a graph representing a first axis coefficient in a two-dimensional scatter diagram of a feature, obtained by linear discriminant analysis of a cell nucleus form performed with parameters representing a feature about the outer shape of a cell nucleus;
FIG. 24B is a graph representing a second axis coefficient in a two-dimensional scatter diagram of a feature, obtained by linear discriminant analysis of a cell nucleus form performed with parameters representing a feature about the outer shape of a cell nucleus;
FIG. 25A is a graph representing distribution states of features of a cell nucleus of DLBCL and a cell nucleus of FL in a first axis direction, in the case of linear discriminant analysis performed with parameters representing a feature about an outer shape;
FIG. 25B is a graph representing the first axis coefficient values in the case of FIG. 25A;
FIG. 25C is a graph representing distribution states of features of a cell nucleus of DLBCL and a cell nucleus of Reactive in a first axis direction, in the case of linear discriminant analysis performed with parameters representing a feature about an outer shape;
FIG. 25D is a graph representing the first axis coefficient values in the case of FIG. 25C;
FIG. 25E is a graph representing distribution states of features of a cell nucleus of FL and a cell nucleus of Reactive in a first axis direction, in the case of linear discriminant analysis performed with parameters representing a feature about an outer shape;
FIG. 25F is a graph representing the first axis coefficient values in the case of FIG. 25E;
FIG. 26A is a two-dimensional scatter diagram representing a distribution of a feature of a cell nucleus of DLBCL, digitized in the diagnosis device of Embodiment 1 of the present disclosure;
FIG. 26B is a two-dimensional scatter diagram representing a distribution of a feature of a cell nucleus of FL, digitized in the diagnosis device of Embodiment 1 of the present disclosure;
FIG. 26C is a two-dimensional scatter diagram representing a distribution of a feature of a cell nucleus of Reactive, digitized in the diagnosis device of Embodiment 1 of the present disclosure;
FIG. 26D is a diagram representing a probability density distribution of a feature of a cell nucleus of DLBCL;
FIG. 26E is a diagram representing a probability density distribution of a feature of a cell nucleus of FL; and
FIG. 26F is a diagram representing a probability density distribution of a feature of a cell nucleus of Reactive.

### Description of Embodiments

Hereinafter, the embodiments of the present disclosure are described with reference to the drawings. The same or similar member is marked with the same symbol in each of the drawings.

### Embodiment 1.

First, Embodiment 1 of the present disclosure is described. A diagnosis device as an information-processing device according to the present embodiment performs a diagnosis of malignant lymphoma. Malignant lymphoma is one of lymphatic tumors. Malignant lymphoma is broadly divided to Hodgkin's lymphoma and non-Hodgkin's lymphoma. In Japan, Hodkin's lymphoma occupies about 5.9% of malignant lymphoma. Malignant lymphoma is mostly non-Hodgkin's lymphoma. Non-Hodgkin's lymphoma is broadly divided to B cell tumor and T/NK cell tumor, and B cell tumors include various classes (subtypes of disease) such as diffuse large-cell B cell lymphoma (DLBCL), follicular lymphoma (FL), MALT lymphoma, small lymphocytic lymphoma, chronic lymphatic leukemia, mantle cell lymphoma, and Nurkitt lymphoma. The number of classes of malignant lymphoma is more than 100. The diagnosis device according to the present embodiment estimates information on the malignant lymphoma of a patient, for example, the presence/absence of suffering from malignant lymphoma, the class, the progress stage, and the like of malignant lymphoma.

The diagnosis device is constructed on a computer. In other words, the diagnosis device is an information-processing device in which information processing by software is specifically realized with a hardware source.

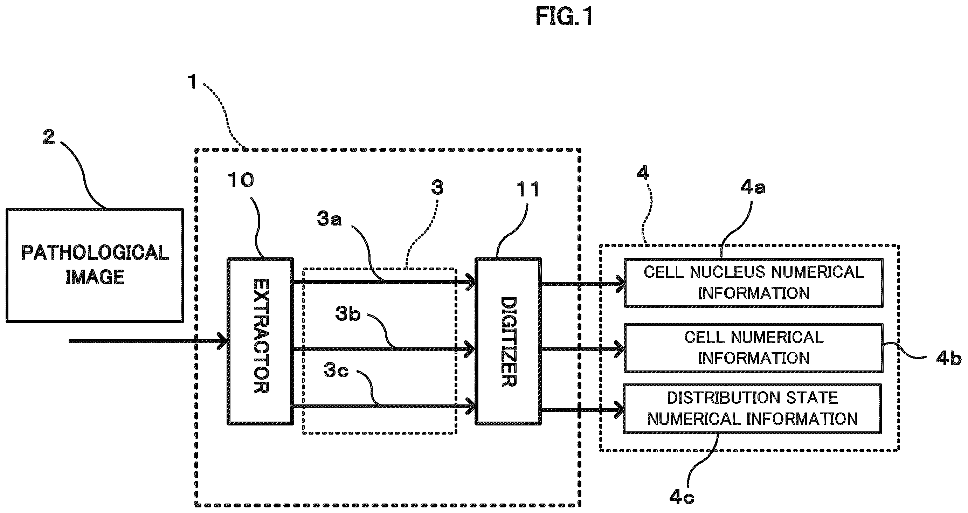

As illustrated in FIG. 1, a pathological image 2 is input in a diagnosis device 1. The pathological image 2 is an image where a plurality of cells including a lymphoid cell is photographed. Examples of the lymphoid cell include a T cell, a B cell, and an NK cell. The lymphoid cell is collected from, for example, a lymph node. The cells photographed in the pathological image 2 include, in addition to the lymphoid cell, various cells. Examples of such cell C include a vascular endothelial cell, a mesenchymal cell including a fibroblast cell, a blood cell other than a lymphoid cell, including histiocyte, an epithelial cell, or a neural cell.

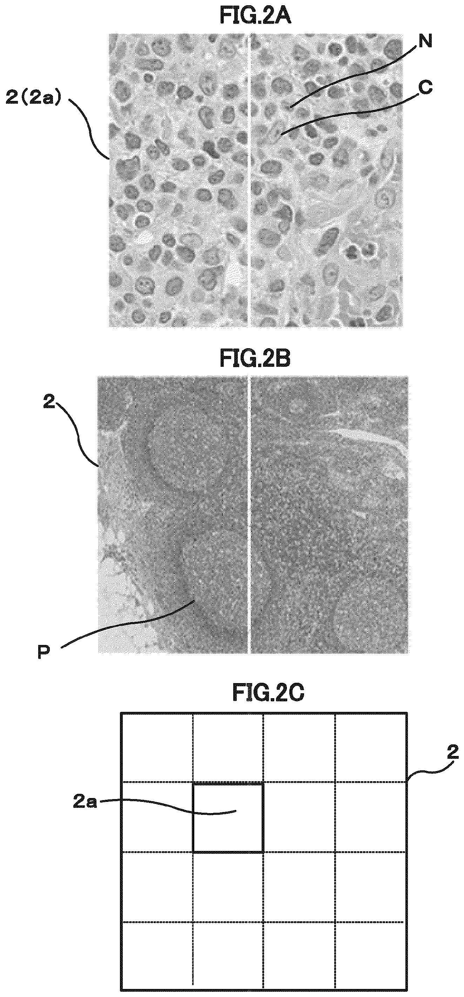

The pathological image 2 is an image of cells stained (for example, HE (Hematoxylin-Eosin) stained). The pathological image 2 has a resolution at which an individual cell nucleus N and an individual cell C are recognizable, for example, as illustrated in FIG. 2A. Furthermore, the pathological image 2 has a range in which a distribution state of cell C is recognizable, for example, as illustrated in FIG. 2B. In the present embodiment, the pathological image 2 (2a) in FIG. 2A can be one portion of a pathological image 2 illustrated in FIG. 2B, as illustrated in FIG. 2C. As illustrated in FIG. 2C, the pathological image 2 is divided to a plurality of pathological images 2a, and the pathological images 2a obtained by such division can be sequentially read by the diagnosis device 1. In other words, the pathological image 2 is an image obtained by imaging a range in which a feature of a distribution state of cell C is recognizable, at a resolution at which features of an individual cell nucleus N and an individual cell C are recognizable.

The diagnosis device 1 performs image processing of the pathological image 2 read, and outputs numerical information 4. The numerical information 4 is classified to cell nucleus numerical information 4a, cell numerical information 4b, and distribution state numerical information 4c. The cell nucleus numerical information 4a is information obtained by digitization of a feature of an individual cell nucleus N photographed in the pathological image 2. The cell numerical information 4b is information obtained by digitization of a feature of an individual cell C photographed in the pathological image 2. The distribution state numerical information 4c is information obtained by digitization of a feature of a distribution state of cell C photographed in the pathological image 2. Such numerical information 4 does not collectively represent features of a plurality of cell nuclei N and a plurality of cells C, but represents features of an individual cell nucleus N and an individual cell C.

A feature of an individual cell nucleus N is about the size, shape or color of the cell nucleus. A feature of an individual cell C is about the size, shape or color of such cell C. A feature about a distribution state of cell C is a feature about uniformity of a plurality of cell C distributions. For example, when the same types of cells C are aggregated in a certain region and distributed so as to form a special pattern, such a feature includes the size and shape of the pattern, the confluency of cells C in the pattern, and a distribution of the pattern itself. Such a feature includes a wide variety of items, and the numerical information 4 (4a,4b, and 4c) output from the diagnosis device 1 serves as a numerical value group in which an aggregation of numerical values calculated with respect to each of the items is collected. The numerical information 4 (4a,4b, and 4c) serves as a diagnostic index objectively representing features about a cell nucleus N, cell C and a distribution state of cell C in the pathological image 2. This cell C is not limited to a lymphoid cell. For example, a vascular endothelial cell, a mesenchymal cell including a fibroblast cell, a blood cell other than a lymphoid cell, including histiocyte, an epithelial cell, or a neural cell is also a subject of feature digitization. The reason is because cell C other than a lymphoid cell is also affected by malignant lymphoma.

The configuration of the diagnosis device 1 is described in more detail. The diagnosis device 1 includes an extractor 10 and a digitizer 11, as illustrated in FIG. 1. The extractor 10 extracts an individual cell nucleus N, an individual cell C and a distribution state of cell C from the pathological image 2 read. A collection of an individual cell nucleus N, an individual cell C and a distribution state of cell C extracted is extraction information 3. The digitizer 11 digitizes a feature of an individual cell nucleus N, a feature of an individual cell C and a feature of a distribution state of cell C, based on an individual cell nucleus N, an individual cell C and a distribution state of cell C extracted by the extractor 10. A collection of those digitized is the numerical information 4.

The extractor 10 is described in more detail. The extractor 10 performs processing that image-processes the pathological image 2 input to thereby differentiate a cell nucleus N of each cell C photographed in the pathological image 2 and other region. After this processing, the extractor 10 outputs cell nucleus extraction information 3a as part of the extraction information 3. The cell nucleus extraction information 3a is information where an image of a cell nucleus N of an individual cell C and other image are distinguishable in the pathological image 2. The extractor 10 performs processing that image processes the pathological image 2 input to thereby differentiate cell C photographed in the pathological image 2 and other region. After this processing, the extractor 10 outputs cell extraction information 3b as part of the extraction information 3. The cell extraction information 3b is information where an image of an individual cell C and other image are distinguishable in the pathological image 2. Furthermore, the extractor 10 detects the type and position information of an individual cell C extracted, extracts a distribution state of cell C based on the type and position information of cell C, and generates distribution state extraction information 3c. The distribution state extraction information 3c includes an aggregate pattern P of the same types of cells C in the pathological image 2.

The digitizer 11 is described in more detail. The digitizer 11 performs image processing of an image of an individual cell nucleus N based on the cell nucleus extraction information 3a, to digitize a feature about the size, shape or color of an individual cell nucleus N and generate cell nucleus numerical information 4a as a numerical value group including numerical values with respect to an individual cell nucleus N and with respect to a feature of the cell nucleus. The digitizer 11 also performs image processing of an image of an individual cell C based on the cell extraction information 3b, to digitize a feature about the size, shape or color of an individual cell C and generate cell numerical information 4b as a numerical value group including numerical values with respect to cell C and with respect to a feature of the cell. Furthermore, the digitizer 11 performs image processing of a plurality of cells C extracted, based on the distribution state extraction information 3c, to digitize a feature about a distribution state of such cells C and generate distribution state numerical information 4c including numerical values with respect to such each cell C and with respect to a feature of a distribution state of such cells C, for example, with respect to an aggregate pattern P.

The cell nucleus numerical information 4a where a feature about the size or shape of a cell nucleus N is digitized includes actually measured values of not only the area of a cell nucleus N, but also the profile length of a cell nucleus N, the width and height of the minimum rectangle receiving an object region of a cell nucleus N, the longer axis, the shorter axis, the angle between the longer axis (principal axis) and the shorter axis (accessory axis), and the roundness of an approximate ellipse, the point-to-point distance (maximum distance, minimum distance) on an object periphery, the aspect ratio, the degree of circularity and the degree of projection of an approximate ellipse, and the like. The approximate ellipse here is an ellipse lowest in difference from the profile of a cell. The roundness is represented by 4π × area/(profile length)². A numerical value of 1.0 means a true circle, and a value closer to 0 means a more elongated shape. The degree of circularity is represented by 4 × area/π/(principal axis length)². The point-to-point distance on an object periphery is a distance between two portions assumed to be mutually opposite in a profile, and the maximum distance is a distance between portions farthest from each other and the minimum distance is a distance between portions nearest to each other, namely, depressed portions of a cell nucleus N.

The cell nucleus numerical information 4a where a feature about the color of a cell nucleus N is digitized includes actually measured values of the average value, the standard deviation, the mode value, the minimum value, the maximum value, the sum, the median value, the third-moment, the fourth-moment, and the percentage of the object region brightness value threshold in the entire image, of brightness value in a cell nucleus N, and the like. The third-moment and the fourth-moment are each an index representing the strain relative to the normal distribution of a brightness value distribution. The third-moment is zero when a brightness value histogram distribution is bilaterally symmetric. The third-moment is negative when the distribution is left tailed, and the third-moment is positive when the distribution is right tailed. The fourth-moment is zero when a brightness value histogram distribution is the normal distribution, the fourth-moment is negative when the distribution is broader, and the fourth-moment is positive when the distribution exhibits a sharper peak. The information may also include, in addition to a feature about brightness value, features about the chromaticness and hue of a cell nucleus N. Numerical information 4 obtained by digitization of a feature about the size, shape or color of an individual cell nucleus N is output as cell nucleus numerical information 4a, from the digitizer 11. The cell nucleus numerical information 4a can here include values obtained by normalization of the above actually measured values with respect to the size, shape and color of a cell nucleus N, for example, by use of statistics of each actually measured value ever acquired and determined in advance from a pathological image or the like, for example, the average value, standard deviation and dispersion of actually measured values with respect to a plurality of cell nuclei N.

The cell numerical information 4b where a feature about the size or shape of cell C is digitized includes measured values of not only the area of cell C, the profile length of cell C, the width and height of the minimum rectangle receiving an object region of cell C, the longer axis, the shorter axis, the angle between the longer axis (principal axis) and the shorter axis (accessory axis), and the roundness of an approximate ellipse, the point-to-point distances (maximum distance, minimum distance) on an object periphery, the aspect ratio, the degree of circularity and the degree of projection of an approximate ellipse, and the like.

The cell numerical information 4b where a feature about the color of cell C is digitized includes actually measured values of the average value, the standard deviation, the mode value, the minimum value, the maximum value, the sum, the median value, the third-moment, the fourth-moment, and the percentage of the object region brightness value threshold in the entire image, of brightness value in cell C. The third-moment and the fourth-moment are each an index representing the strain relative to the normal distribution of a brightness value distribution. The information may also include, in addition to a feature about brightness value, features about the chromaticness and hue of cell C. Numerical information 4 obtained by digitization of a feature about the size, shape or color of an individual cell C is output as cell numerical information 4b, from the digitizer 11. The cell numerical information 4b can here include values obtained by normalization of the above actually measured values with respect to the size, shape and color of cell C, for example, by use of statistics of each actually measured value with respect to cell C, ever acquired and determined in advance from a pathological image or the like, for example, the average value, standard deviation and dispersion of actually measured values with respect to a plurality of cells C.

An aggregate pattern P of the same types of cells C, obtained from the classes and position coordinates of a plurality of cells C, serves as a feature of a distribution state of cell C. Actually measured values of the presence/absence of such an aggregate pattern P of the same types, the type, shape and size of the same types of cells forming an aggregation, the number of aggregations, the confluency in an aggregation, and the like are included in the numerical information where a feature of a distribution state of cell C is digitized. Such numerical information is output as distribution state numerical information 4c, from the digitizer 11. The distribution state numerical information 4c can here include values obtained by normalization of the above actually measured values with respect to a distribution state of cell C, for example, by use of statistics of each actually measured value with respect to a distribution state, ever acquired and determined in advance from a pathological image or the like, for example, the average value, standard deviation and dispersion of actually measured values with respect to distribution states of cells C in a plurality of pathological images.

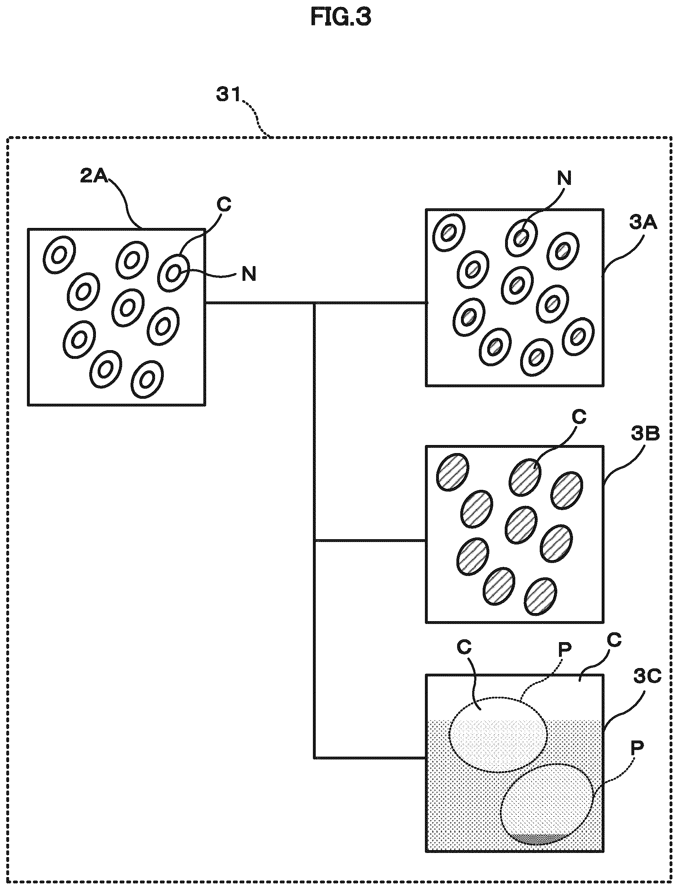

The extractor 10 is a machine learning tool that performs image processing by machine learning. The extractor 10 performs machine learning before actual input of a pathological image 2 of a diagnosis target. A sample image 2A as a sample of the pathological image 2 is prepared for machine learning by the extractor 10, as illustrated in FIG. 3. The sample image 2A is a pathological image representing a plurality of cells C. The sample image 2A is associated with sample images 3A, 3B, and 3C. The sample image 3A is an image where an individual cell nucleus N of cell C photographed in the image is manually extracted by a clinician. The sample image 3B is an image where an individual cell C photographed in the pathological image 2 is manually extracted by a clinician. The sample image 3C is an image where an aggregate pattern P of the same types of cells C photographed in the pathological image 2 is manually extracted by a clinician. The sample image 3C is an image representing a distribution state of cell C manually extracted from the sample image 2A.

The foregoing is summarized as follows. The sample images 3A to 3C are respectively images representing an individual cell nucleus N, an individual cell C and a distribution state of cell C (aggregate pattern P) manually extracted from the sample image 2A. A combination of the sample images 2A and 3A to 3C serves as sample data representing a relationship between the sample image 2A as a sample of the pathological image 2, and an individual cell nucleus N, an individual cell C and a distribution state of cell C manually extracted from the sample image 2A.

The extractor 10 performs machine learning with a sample image where a cell nucleus N and cell C are manually extracted by a clinician. The extractor 10 extracts an individual cell nucleus N, an individual cell C and a distribution state of cell C from the pathological image 2, based on a result of the machine learning. The sample images 3A to 3C each serve as teacher data, and thus this machine learning is supervised learning. The sample images 3A to 3C are each an image where an individual cell nucleus N, an individual cell C and a distribution state of cell C are extracted by a clinician. Thus, know-how of extraction by a clinician is taken into the extractor 10 by the machine learning, and the extractor 10 can perform image processing reflecting the know-how by a clinician, to extract an individual cell nucleus N, an individual cell C and a distribution state of cell C.

Such a machine learning tool here used can be, for example, a learning tool performing deep learning of an image. Such a learning tool here used can be, for example, one into which a convolution neural network is incorporated. In a case where the sample image 2A illustrated in FIG. 3 is input to such a convolution neural network, for example, with an error backward propagation method, the machine learning is performed so that an individual cell nucleus N is extracted and the cell nucleus extraction information 3a is generated as in the sample image 3A, an individual cell C is extracted and the cell extraction information 3b is generated as in the sample image 3B, and a distribution state of cell C is extracted and the distribution state extraction information 3c is generated as in the sample image 3C.

In fact, a plurality of sample data 31 with a combination of the sample images 2A and 3A to 3C is prepared. For example, a plurality of combinations of sample data 31 of a subject not suffering from malignant lymphoma, and a plurality of combinations of sample data 31 with respect to each class of malignant lymphoma are desirably prepared. As the number of samples with respect to each of the combinations is larger, the accuracy of extraction of an individual cell nucleus N, an individual cell C and a distribution state of cell C with the extractor 10 can be increased.

The digitizer 11 digitizes a feature of an individual cell nucleus N, a feature of an individual cell C and a feature of a distribution state of cell C, correlated with information on malignant lymphoma, based on an individual cell nucleus N, an individual cell C and a distribution state of cell C extracted by the extractor 10. The digitizer 11 calculates cell nucleus numerical information 4a where features about the shape and color of an individual cell nucleus N are digitized based on a cell nucleus N extracted from the pathological image 2. The digitizer 11 calculates cell numerical information 4b where features about the shape and color of an individual cell C are digitized based on cell C extracted from the pathological image 2. Furthermore, the digitizer 11 calculates distribution state numerical information 4c where a feature about a distribution state of cell C is digitized based on a distribution state of cell C extracted from the pathological image 2.

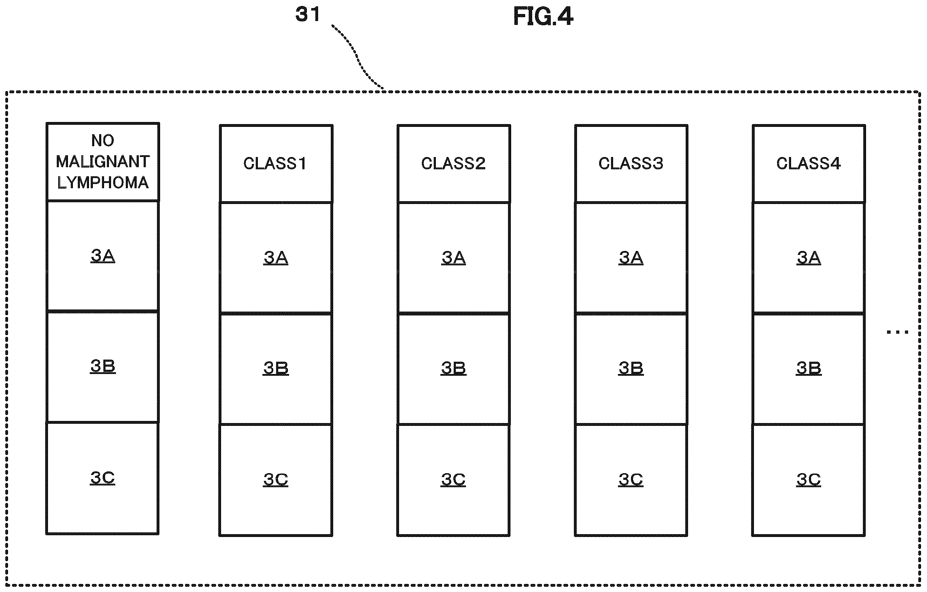

The digitizer 11 can be configured by a machine learning tool. The digitizer 11 performs machine learning before actual input of extraction information 3 obtained from a pathological image 2 of a diagnosis target. Sample data 31 illustrated in FIG. 4 is prepared for this machine learning. As illustrated in FIG. 4, this sample data 31 here prepared includes sample images 3A, 3B, and 3C with respect to a subject not suffering from malignant lymphoma (no malignant lymphoma) and sample images 3A, 3B, and 3C with respect to classes of (classes 1, 2, 3, 4, ... ) of malignant lymphoma. As described above, the sample image 3A corresponds to the cell nucleus extraction information 3a, the sample image 3B corresponds to the cell extraction information 3b, and the sample image 3C corresponds to the distribution state extraction information 3c. Such sample data 31 is data representing a relationship between the sample images 3A to 3C where a feature of an individual cell nucleus N, a feature of an individual cell C and a feature of a distribution state of cell C are extracted, and information on malignant lymphoma, namely, the presence/absence and class of malignant lymphoma.

The digitizer 11 performs machine learning based on the sample data 31 illustrated in FIG. 4. The digitizer 11 narrows a feature of an individual cell nucleus N, a feature of an individual cell C and a feature of a distribution state of cell C, down to a feature to be digitized, which is correlated with malignant lymphoma, by the machine learning.

Specifically, the machine learning allows the digitizer 11 to distinguish a feature of an individual cell nucleus N, a feature of an individual cell C and a feature of a distribution state of cell C, to one where a significant difference is identified depending on the presence/absence of malignant lymphoma and one where no significant difference is identified depending thereon. For example, in a case where any significant difference in size and degree of projection of a cell nucleus N is identified depending on the presence/absence of malignant lymphoma, a feature to be digitized by the digitizer 11 can be narrowed down to information where such a significant difference is identified. In a case where any significant difference in numerical information representing the area and brightness value of cell C is identified depending on the presence/absence of malignant lymphoma, a feature to be digitized by the digitizer 11 can be narrowed down to information where such a significant difference is identified. Furthermore, in a case where any significant difference in aggregate pattern P of a distribution state of cell C is identified depending on the presence/absence of malignant lymphoma, a feature to be digitized by the digitizer 11 can be narrowed down to information where such a significant difference is identified. Such machine learning here adopted can be, for example, a dimension reduction procedure by principle component analysis. Herein, the "narrowing down to" encompasses generating a new feature (feature serving as a principle component) represented by linear coupling or the like of each feature described above, changing the weight of each feature in the new feature, and performing feature weighting.

Such narrowing can be performed to reduce the number of features for calculation of the numerical information 4 by the digitizer 11 and shorten the time taken for the calculation, resulting in an increase in the efficiency of a diagnosis of malignant lymphoma.

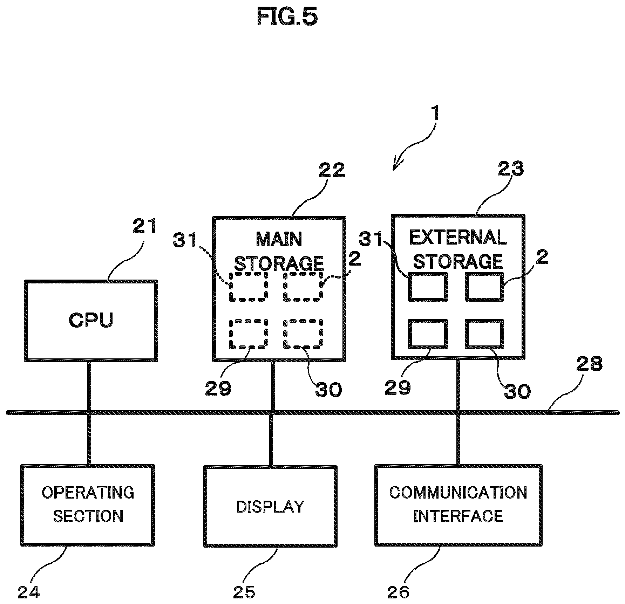

The diagnosis device 1 illustrated in FIG. 1 is realized by, for example, allowing a computer having a hardware configuration illustrated in FIG. 5 to realize a software program. Specifically, the diagnosis device 1 is configured from a central processing unit (CPU) 21 responsible for the control of the entire device, a main storage 22 operating as an operating region or the like of the CPU 21, an external storage 23 storing an operation program and the like of the CPU 21, an operating section 24, a display 25, a communication interface 26, and an internal bus 28 connecting them.

The main storage 22 is configured from, for example, random access memory (RAM). In the main storage 22, a diagnosis program 29 of the CPU 21, a machine learning program 30, sample data 31, and a pathological image 2 are loaded from the external storage 23. The main storage 22 is also used as an operating region (temporary storage region of data) of the CPU 21.

The external storage 23 is configured from a non-volatile memory such as a flash memory or a hard disc. The external storage 23 stores a diagnosis program 29 to be executed by the CPU 21, and a machine learning program 30, in advance. The external storage 23 further stores sample data 31, and a pathological image 2 collected from a living organism as a patient of a diagnosis target. The sample data 31 includes, as described above, data of a combination of the sample images 2A and 3A to 3C (see FIG. 3), and data of a combination of the sample images 3A to 3C (see FIG. 4), in which the presence/absence and class of malignant lymphoma are determined.

The operating section 24 is configured from devices such as a keyboard and a mouse, and an interface device connecting these devices to the internal bus 28.

The display 25 is configured from a display device such as a cathode ray tube (CRT) or a liquid crystal monitor.

The communication interface 26 is an interface performing sending and receiving of data to and from external equipment. The sample data 31 or the pathological image 2 is sent and received via the communication interface 26.

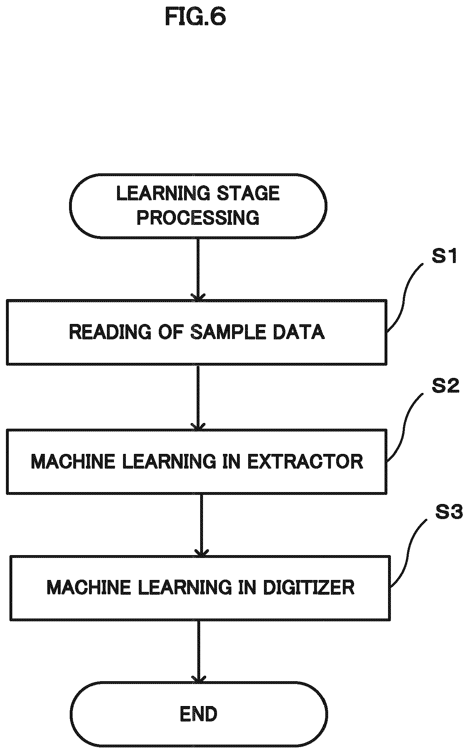

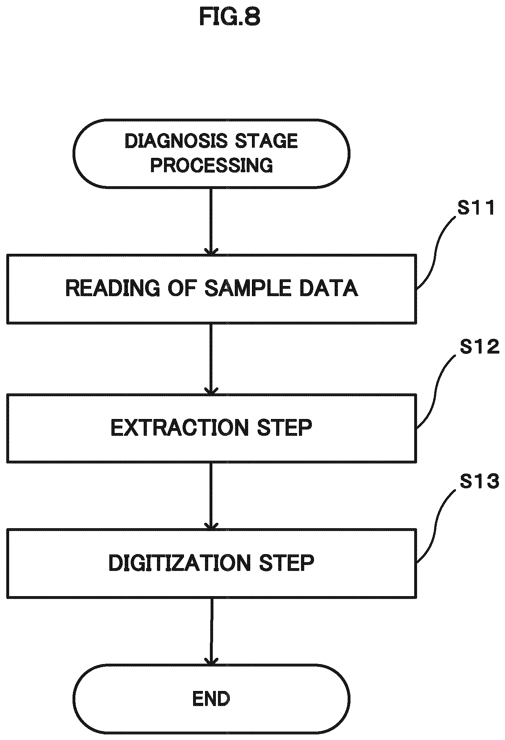

Next, an information acquisition method realized by operating of the diagnosis device 1 according to the present embodiment, namely, processing to be executed by the diagnosis device 1 is described. The processing to be executed by the diagnosis device 1 is divided to learning stage processing illustrated in FIG. 6 and diagnosis stage processing illustrated in FIG. 8.

First, the learning stage processing is described. The processing at the learning stage is executed by allowing the machine learning program 30 to be read from the external storage 23 to the main storage 22 and allowing the CPU 21 to execute the machine learning program 30.

As illustrated in FIG. 6, first, the diagnosis device 1 performs reading of the sample data 31 (Step S1). The sample data 31 is here read from the external storage 23 to the main storage 22.

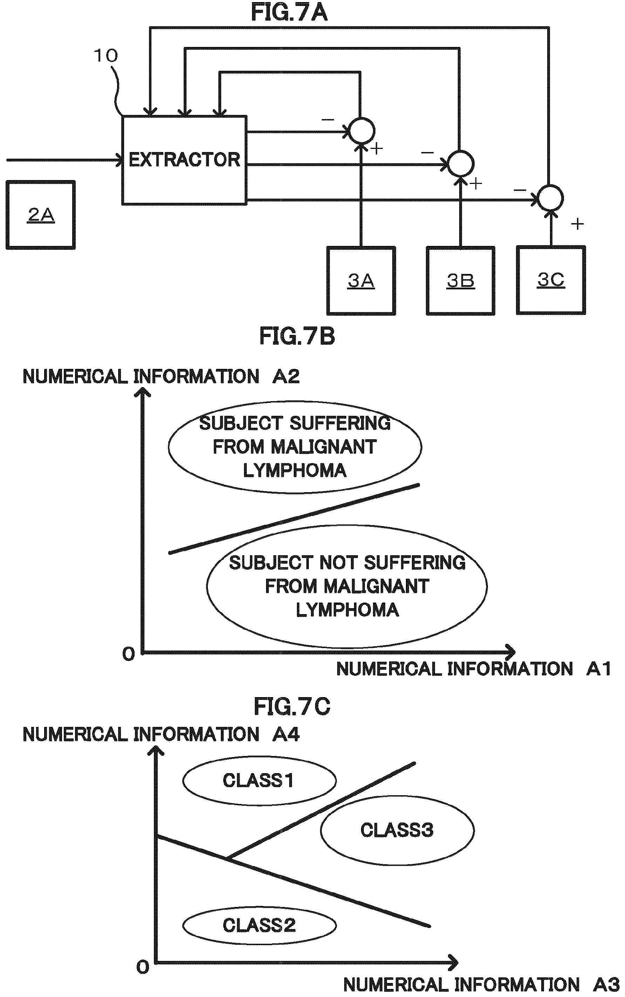

Subsequently, the extractor 10 of the diagnosis device 1 performs machine learning with a combination of the sample images 2A and 3A to 3C (see FIG. 3) in the sample data 31 (Step S2). The machine learning allows an internal parameter to be adjusted so that the extractor 10 outputs the sample images 3A to 3C in the case of input of the sample image 2A, for example, as illustrated in FIG. 7A. In other words, an internal parameter is adjusted so as to decrease the respective deltas between an image corresponding to the cell nucleus extraction information 3a, an image corresponding to the cell extraction information 3b and an image corresponding to the distribution state extraction information 3c due to output from the extractor 10, and the sample image 3A, the sample image 3B and the sample image 3C. The internal parameter is used in the diagnosis program 29 described below, and thus is stored in the main storage 22 and the external storage 23.

Subsequently, the digitizer 11 of the diagnosis device 1 performs machine learning with a combination of the sample images 3A to 3C (see FIG. 4), in which the presence/absence and class of malignant lymphoma are determined, in the sample data 31 (Step S3). The combination of the sample images 3A to 3C, here used, can be the cell nucleus extraction information 3a, the cell extraction information 3b and the distribution state extraction information 3c output from the extractor 10 in which the machine learning is completed.

The machine learning allows the digitizer 11 to sequentially input the sample data 31 as the combination of the sample images 3A to 3C illustrated in FIG. 4 and generate, for example, numerical information A1 and A2 on features of a cell nucleus N, cell C and a distribution state of cell C, as illustrated in FIG. 7B. In a case where respective distributions with respect to a subject not suffering from malignant lymphoma and a subject suffering from malignant lymphoma can be distinguished as illustrated in FIG. 7B, the numerical information A1 and A2 are correlated with the presence/absence of malignant lymphoma. As illustrated in FIG. 7C, numerical information A3 and A4 are correlated with the classes (class 1, 2, 3) of malignant lymphoma. The digitizer 11 thus determines whether or not each item of the numerical information 4 has correlativity described above, and narrows an object of calculation down to one having such correlativity. Additionally, the digitizer 11 determines whether or not each item of the numerical information 4 has correlativity described above, and narrows an object of calculation down to one having such correlativity. A feature extracted is stored in the main storage 22 or the external storage 23. After completion of Step S3, the diagnosis device 1 terminates the learning stage processing.

Next, the diagnosis stage processing is described. The processing at the diagnosis stage is executed by allowing the diagnosis program 29 to be read from the external storage 23 to the main storage 22 and allowing the CPU 21 to execute the diagnosis program 29.

As illustrated in FIG. 8, first, the diagnosis device 1 performs reading of the pathological image 2 (Step S11). The pathological image 2 is here read from the external storage 23 to the main storage 22.

Subsequently, the extractor 10 of the diagnosis device 1 performs an extraction step of extracting an individual cell nucleus N, an individual cell C and a distribution state of cell C from the pathological image 2 (Step S12). The internal parameter determined in Step S2 (see FIG. 6) at the learning stage is set, and an individual cell nucleus N, an individual cell C and a distribution state of cell C are extracted from the pathological image 2, according to the internal parameter. Thus, an individual cell nucleus N, an individual cell C and a distribution state of cell C are extracted from the pathological image 2, according to the result of the machine learning.

Subsequently, the digitizer 11 of the diagnosis device 1 performs a digitization step of digitizing a feature of an individual cell nucleus N, a feature of an individual cell C and a feature of a distribution state of cell C based on a cell nucleus N and a cell C extracted by the extractor 10 (Step S13). The numerical information 4 is here calculated with respect to only a feature of an individual cell nucleus N, a feature of an individual cell C and a feature of a distribution state of cell C extracted in Step S3 (see FIG. 6) at the learning stage. After completion of Step S13, the diagnosis device 1 terminates the learning stage processing.

As described above in detail, the diagnosis device 1 according to the present embodiment digitizes features of an individual cell nucleus N, an individual cell C, a distribution state of cell C in a plurality of cells including a lymphoid cell C, variously changed depending on suffering from malignant lymphoma. An individual cell nucleus N, an individual cell C and a distribution state of cell C are changed depending on suffering from malignant lymphoma, and are different in features of change with respect to each class. Features digitized, of an individual cell nucleus N, an individual cell C and a distribution state thereof, serve as an objective and unified index in a diagnosis of malignant lymphoma by a physician. Accordingly, the present embodiment can provide a unified diagnostic index for diagnosing malignant lymphoma.

More specifically, the diagnosis device 1 can allow for digitization of a feature of an individual cell nucleus N and a feature of an individual cell C of various lymphoid cells or other cells, without any loss of these features, and thus can fully pick up various influences of malignant lymphoma on lymphoid cells or other cells and acquire information thereon. A conventional cell image diagnosis of malignant lymphoma has been performed along with the diagnosis skill acquired by a pathologist himself/herself. In this case, the pathologist has performed such a diagnosis by sensory catching of features about an individual cell nucleus N, an individual cell C and a distribution state of cell C, and thus different determinations have been sometimes made even with the same pathological image 2, depending on each pathologist. On the contrary, the diagnosis device 1 allows for digitization of features about an individual cell nucleus N, an individual cell C and a distribution state of cell C. Such numerical values can be each used for an objective diagnostic index of malignant lymphoma. In other words, the diagnosis device 1 can fully pick up features about an individual cell nucleus N, an individual cell C and a distribution state of cell C to obtain an objective and accurate diagnostic index for a diagnosis of malignant lymphoma.

In the present embodiment, the digitizer 11 is to perform the machine learning to thereby narrow a feature of an individual cell nucleus N, a feature of an individual cell C and a feature of a distribution state of cell C down to the numerical information 4 to be digitized. Thus, information to be digitized can be narrowed down to significant information correlated with malignant lymphoma, and a more objective and accurate diagnosis of malignant lymphoma can be made.

However, the present disclosure is not limited thereto. For example, the digitizer 11 may be set so that a feature of the numerical information 4 determined from a plurality of combinations of the sample images 3A to 3C, in which a significant difference depending on the presence/absence of malignant lymphoma and a significant difference depending on the class of malignant lymphoma are identified, is extracted from features about an individual cell nucleus N, an individual cell C and a distribution state of cell C, in advance, by another information-processing device, and only the numerical information 4 on the feature determined is calculated.

The ability to process an enormous amount of information and link it to a diagnosis of malignant lymphoma is needed for full picking up of an individual cell nucleus N, an individual cell C and a distribution state of cell C, and then a diagnosis. The diagnosis device 1 according to the present embodiment can realize processing for linkage of an enormous amount of information to an accurate diagnosis of malignant lymphoma, by loading a machine learning tool and reflecting the skill of a clinician to information processing in the machine learning tool. The machine learning method exemplified in the present embodiment is merely illustrative, and other various methods can be each adopted. A statistical method such as regression analysis can also be used for any portion that can be performed even without the machine learning.

### Embodiment 2.

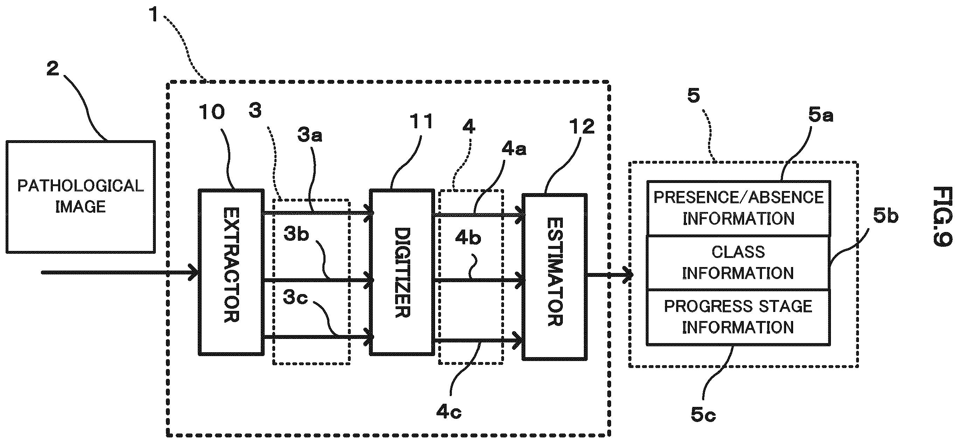

Next, Embodiment 2 of the present disclosure is described. The configuration and operation are described in Embodiment 1 described above, where a cell nucleus N and cell C are extracted from a pathological image 2 of a diagnosis target and a feature of an individual cell nucleus N, a feature of an individual cell C and a feature of a distribution state of cell C are digitized based on a cell nucleus N and a cell C extracted. A diagnosis device 1 according to the present embodiment is different from the diagnosis device 1 according to Embodiment 1 described above in that an estimator 12 is included in addition to an extractor 10 and a digitizer 11, as illustrated in FIG. 9. The configurations of the extractor 10 and the digitizer 11 are the same as in Embodiment 1 described above.

The estimator 12 estimates information on malignant lymphoma based on a feature of an individual cell nucleus N, a feature of an individual cell C and a feature of a distribution state of cell C, digitized by the digitizer 11. The estimator 12 outputs information on malignant lymphoma as estimated information 5. The information on malignant lymphoma includes presence/absence information 5a representing the presence/absence of suffering from malignant lymphoma, class information 5b representing the class of malignant lymphoma, and progress stage information 5c representing the progress stage of malignant lymphoma.

The estimator 12 is a machine learning tool that performs image processing by machine learning. The estimator 12 performs machine learning before the actual input of a pathological image 2 of a diagnosis target.

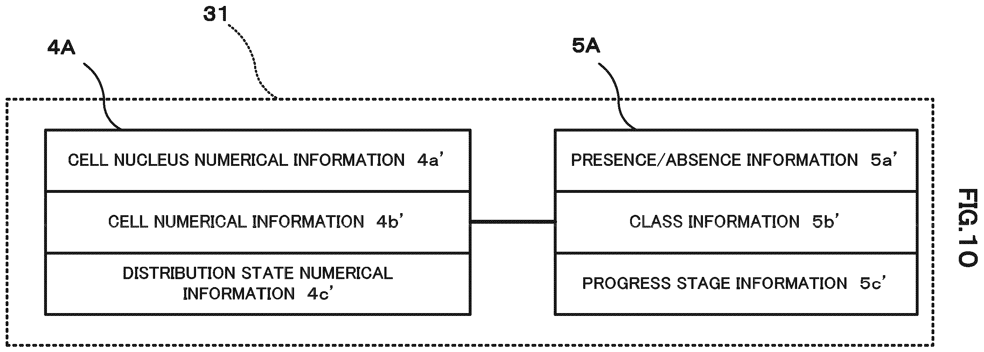

The machine learning is performed with sample data 31 illustrated in FIG. 10. As illustrated in FIG. 10, the sample data 31 is data including a combination of numerical information 4A corresponding to the numerical information 4 as the input data of the estimator 12 and estimated information 5A corresponding to the estimated information 5 of the output data of the estimator 12.

The numerical information 4A includes numerical information (cell nucleus numerical information 4a') on a feature of a cell nucleus N, numerical information (cell numerical information 4b') on a feature of cell C and numerical information (distribution state numerical information 4c') on a feature of a distribution state of cell C, of a certain patient. The estimated information 5A includes a combination with information 5a' on the presence/absence of malignant lymphoma, information 5b' on the class of malignant lymphoma and information 5c' on the progress stage of malignant lymphoma. Machine learning by the estimator 12 is performed so that, if the cell nucleus numerical information 4a', the cell numerical information 4b' and the distribution state numerical information 4c' of the numerical information 4A are input, the presence/absence information 5a', the class information 5b' and the progress stage information 5c' of the estimated information 5A are output.

Examples of such a machine learning tool that can be here applied include various learning tools such as a learning tool for classification as supervised learning, in addition to a learning tool for deep learning. Examples of such a learning tool that can be here applied include a support vector machine, a decision tree learning tool, and an ensemble learning tool. The estimator 12 estimates information on malignant lymphoma from a feature of an individual cell nucleus N and features of an individual cell C and a distribution state of cell C, digitized by the digitizer 11, based on the result of the machine learning.

The hardware configuration of the diagnosis device 1 according to the present embodiment is the same as in Embodiment 1 described above (FIG. 5).

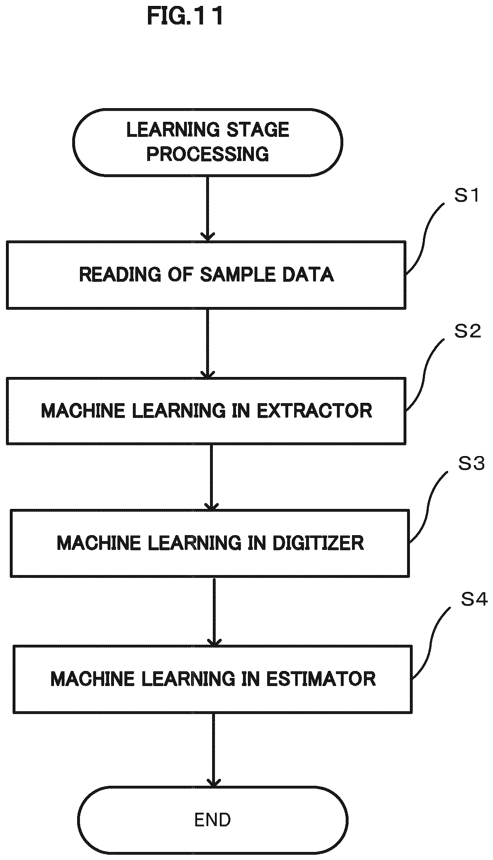

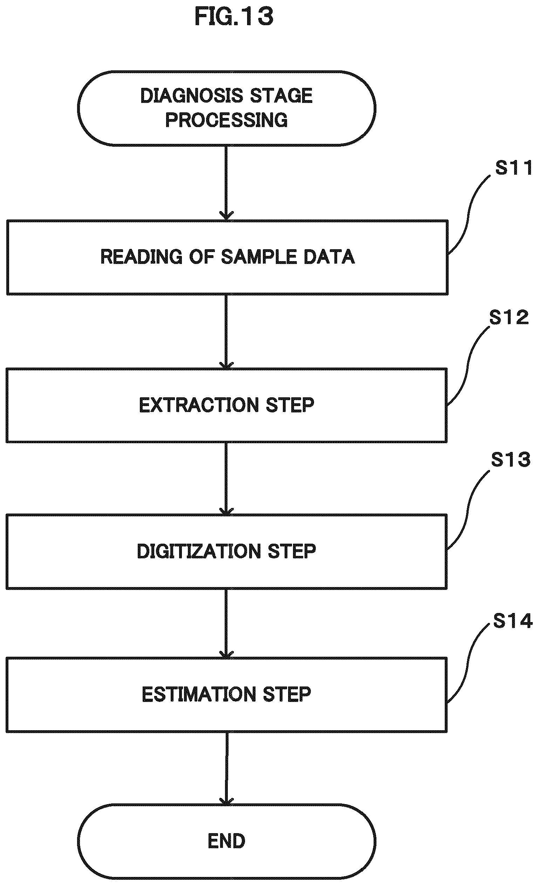

Next, processing realizing an information acquisition method to be executed by the diagnosis device 1 according to the present embodiment is described. The processing to be performed by the diagnosis device 1 is divided to learning stage processing illustrated in FIG. 11 and diagnosis stage processing illustrated in FIG. 13.

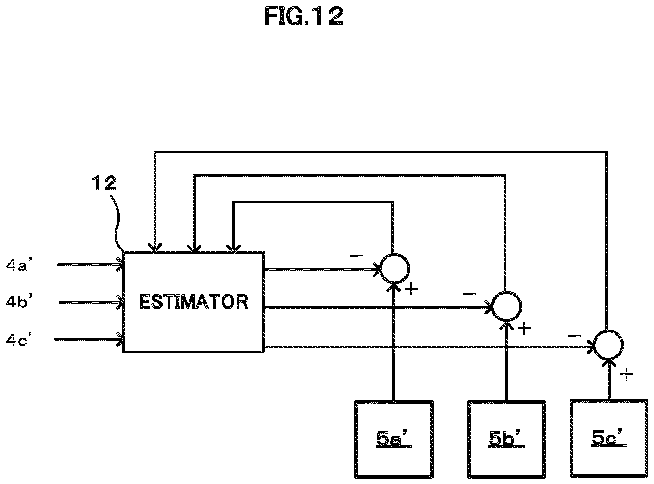

As in Embodiment 1 described above, the processing at the learning stage is executed by reading the machine learning program 30 from the external storage 23 to the main storage 22 and executing the machine learning program 30 in the CPU 21. As illustrated in FIG. 11, the diagnosis device 1 performs reading of sample data (Step S1), machine learning by the extractor 10 (Step S2), machine learning by the digitizer 11 (Step S3), and thereafter machine learning by the estimator 12 (Step S4). In Step S4, the estimator 12 performs machine learning based on sample data 31 representing a relationship between a feature of an individual cell nucleus N, a feature of an individual cell C and a feature of a distribution state of cell C, and information on malignant lymphoma. As illustrated in FIG. 12, internal parameter adjustment is performed by the estimator 12 so that the delta between the output in input of the cell nucleus numerical information 4a', the cell numerical information 4b' and the distribution state numerical information 4c', and the presence/absence information 5a', the class information 5b' and the progress stage information 5c' is smaller. The cell nucleus numerical information 4a', the cell numerical information 4b' and the distribution state numerical information 4c' here used can be each information output from the digitizer 11 in which machine learning is completed in Step S3.

As in the above embodiment, the diagnosis device 1 performs reading of sample data (Step S11), an extraction step (Step S12) and a digitization step (Step S13), and thereafter an estimation step (Step S14) of estimating information on malignant lymphoma based on a feature of an individual cell nucleus N, a feature of an individual cell C and a feature of a distribution state of cell C, digitized by the digitizer 11, at the diagnosis stage, as illustrated in FIG. 13. Since estimation by the estimator 12 is performed with the internal parameter adjusted by the machine learning in Step S4 at the learning stage, the estimation results can be unified and objective based on the foregoing diagnosis.

According to the diagnosis device 1 of the present embodiment, information on malignant lymphoma, namely, the presence/absence, the class and the progress stage of malignant lymphoma can be estimated at a high accuracy based on the numerical information (cell nucleus numerical information 4a) representing a feature of an individual cell nucleus N, the numerical information (cell numerical information 4b) representing a feature of an individual cell C and the numerical information (distribution state numerical information 4c) representing a feature of a distribution state of cell C, digitized by the digitizer 11, and the presence/absence information 5a, the class information 5b and the progress stage information 5c can be obtained.

### Embodiment 3.

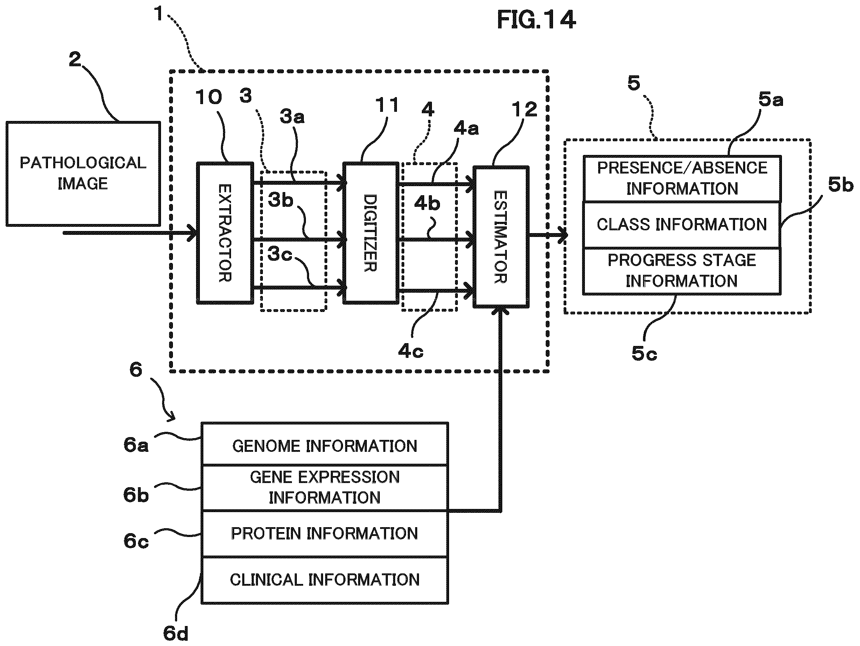

Next, Embodiment 3 of the present disclosure is described. The diagnosis device 1 according to Embodiment 2 described above performs estimation of information on malignant lymphoma based on features of an individual cell nucleus N, an individual cell C and a distribution state of cell C, obtained from the pathological image 2. The diagnosis device 1 according to the present embodiment performs estimation of information on malignant lymphoma, namely, estimated information 5 based on not only numerical information 4 on features of an individual cell nucleus N, an individual cell C and a distribution state of cell C, obtained from a pathological image 2, but also at least one additional information 6 of genome information 6a, gene expression information 6b, protein information 6c or clinical information 6d correlated with malignant lymphoma, as illustrated in FIG. 14.

The genome information 6a is here information on DNA (deoxyribonucleic acid) of the corresponding cell. For example, the genome information 6a includes information on a chromosomal abnormality generated in suffering from malignant lymphoma. For example, chromosomal abnormalities such as t(14:18) of FL, t(11:18) of MALT lymphoma, t(11:14) of mantle cell lymphoma, and t(8:14) of Burkitt lymphoma are identified. The genome information 6a is information representing a genetic abnormality including such a chromosomal abnormality.

The gene expression information 6b is information representing a gene expressed due to malignant lymphoma. For example, expression of genes such as BCL2 of FL, cyclin D1 of mantle cell lymphoma, and MYC of Burkitt lymphoma is identified. The gene expression information 6b is information representing such a cancer-associated gene.

The protein information 6c is information on a protein expressed in a lymphoid cell of malignant lymphoma. The protein information 6c includes information including cell surface markers such as Cluster of Differentiation 3 (CD3), intracytoplasmic CD3ε, CD5, CD45, CD20, CD79a, and CD10, and immunoglobulin (intracytoplasmic immunoglobulin).

The clinical information 6d is information representing patient's history, physical finding, general survey, and pathological tissue diagnosis results. The information includes patient's B symptoms (namely fever, night sweats, and unintentional weight loss), sweet itch, lymph node enlargement, hematological examination (blood count, biochemistry, serum), immunoreaction, interviewing, virus inspection, urinalysis, image inspection (chest X-ray, Computed Tomography (CT)), upper gastrointestinal endoscope, Positron Emission Tomography (PET), scintigraphy, lymph node biopsy, and bone-marrow, pleural effusion, ascites and cerebrospinal fluid examination results.

As illustrated in FIG. 14, not only the numerical information 4, but also the genome information 6a, the gene expression information 6b, the protein information 6c or the clinical information 6d correlated with malignant lymphoma is input to the estimator 12. The estimator 12 estimates information on malignant lymphoma in consideration of not only the numerical information 4, but also the genome information 6a, the gene expression information 6b, the protein information 6c or the clinical information 6d. The estimator 12 performs machine learning based on sample data 31 representing a relationship between a feature of an individual cell nucleus N, a feature of an individual cell C, a feature of a distribution state of cell C and additional information 6, and information on malignant lymphoma. The estimator 12 estimates information on malignant lymphoma based on the result of the machine learning.

Thus, information on malignant lymphoma can be accurately estimated based on not only the pathological image 2, but also examination results of genome, gene expression and protein correlated with other malignant lymphoma, examination results in a medical setting, and a diagnosis result by a physician.

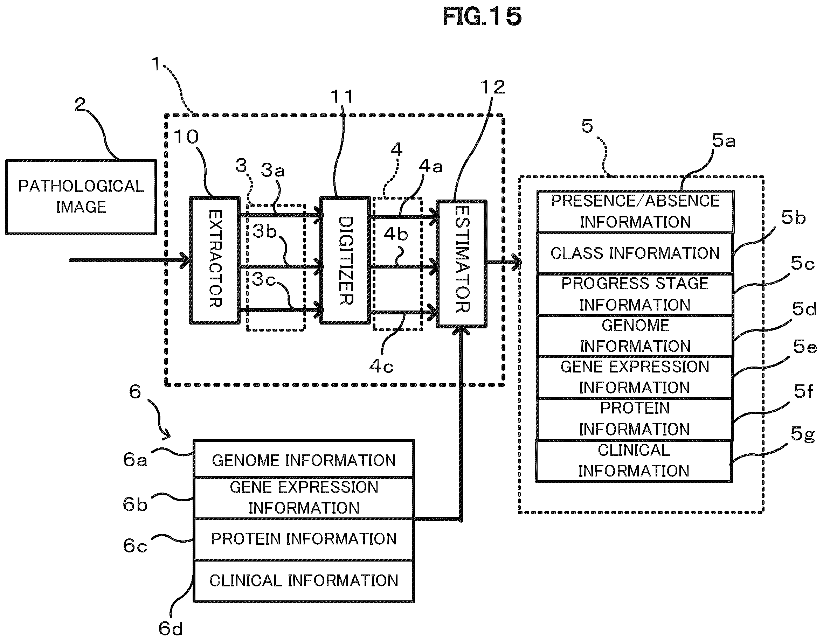

As illustrated in FIG. 15, information on malignant lymphoma may include at least one of the presence/absence information 5a, the class information 5b or the progress stage information 5c of malignant lymphoma, or the genome information 5d, the gene expression information 5e, the protein information 5f or clinical information 5g correlated with malignant lymphoma. For example, information such as "poor prognosis due to finding of correlation with specific gene" or "poor prognosis due to finding of correlation with specific protein" can be output as information on malignant lymphoma. In other words, at least one additional information 6 of the genome information 6a, the gene expression information 6b, the protein information 6c or the clinical information 6d can be input and at least one additional information 5 of genome information 5d, the gene expression information 5e, the protein information 5f or the clinical information 5g can be output by the estimator 12. No overlapping of information may be made between the input and the output.

### Embodiment 4.

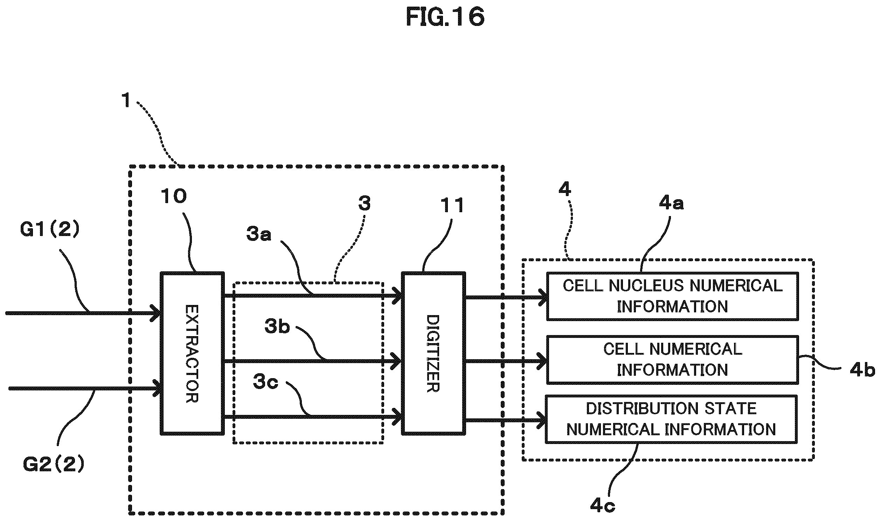

Next, Embodiment 4 of the present disclosure is described. In the diagnosis devices 1 according to Embodiments 1 to 3 described above, the numerical information 4 representing features indicating an individual cell nucleus N, an individual cell C and a distribution state of cell C is calculated based on one pathological image 2, namely, a pathological image 2 that has a range in which a feature of a distribution state of cell C is recognizable and that is taken at a resolution at which features of an individual cell nucleus N and an individual cell C are recognizable. A first image G1 and a second image G2 are used for the pathological image 2 in the diagnosis device 1 according to the present embodiment, as illustrated in FIG. 16. The first image G1 is an image for recognition of an individual cell nucleus N and an individual cell C. The second image G2 is an image for recognition of a distribution state of cell C. The first image G1 here used can be, for example, a pathological image 2 illustrated in FIG. 2C, and the second image G2 here used can be, for example, one pathological image 2a illustrated in FIG. 2C. The first image G1 and the second image G2 are thus required to be respectively images of cells collected from the same living organism.

The extractor 10 not only extracts an individual cell nucleus N and an individual cell C from the first image G1 in which an individual cell nucleus N and an individual cell C are recognizable, but also extracts an aggregate pattern P of the same types of cells C from the second image G2 as the range in which a feature of a distribution state of cell C is recognizable.

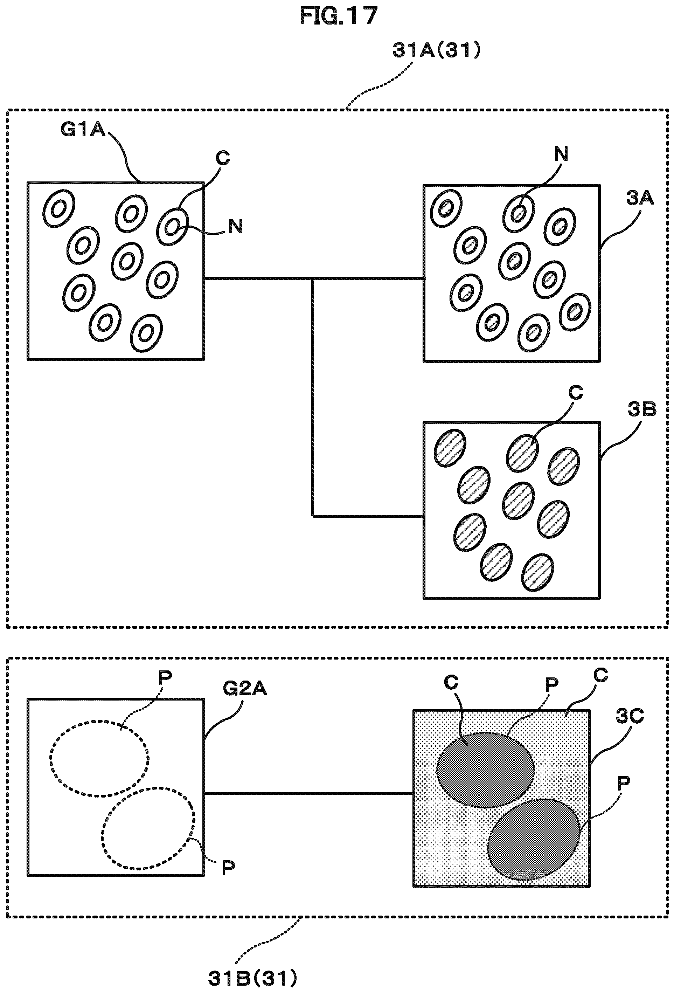

The extractor 10 performs machine learning in the same manner as described in Embodiments 1 to 3 above. The machine learning is performed with sample data 31A and sample data 31B for the sample data 31, as illustrated in FIG. 17.

The sample data 31A is data including a combination of a first sample image G1A corresponding to the first image G1, a sample image 3A in which an individual cell nucleus N is manually extracted from the first sample image G1A and a sample image 3B in which an individual cell C is manually extracted from the first sample image G1A. In other words, the sample data 31A is data representing a relationship between the first sample image G1A corresponding to the first image G1, and an individual cell nucleus N and an individual cell C manually extracted from the first sample image G1A.

The sample data 31B is data including a combination of a second sample image G2A corresponding to the second image G2 and a sample image 3C in which a distribution state of an individual cell C is manually extracted from the second sample image G2A. In other words, the sample data 31B is data representing a relationship between the second sample image G2A corresponding to the second image G2, and a distribution state of an individual cell C manually extracted from the second sample image G2A. The extractor 10 performs machine learning with the sample data 31A and 31B, and not only extracts an individual cell nucleus N and an individual cell C from the first image G1 based on the result of the machine learning, but also extracts a distribution state of cell C from the second image G2.

The machine learning and feature digitization by the digitizer 11, and the machine learning and estimation by the estimator 12 are performed in the same manner as described in Embodiments 1 to 3 above.

As described above, the diagnosis device 1 can extract an individual cell nucleus N, an individual cell C and a distribution state of cell C from the pathological image 2, according to processing reflecting the skill of a pathologist, by the machine learning. The diagnosis device 1 can digitize features about an individual cell nucleus N, an individual cell C and a distribution state of cell C correlated with malignant lymphoma to provide an objective diagnostic index of malignant lymphoma, by the machine learning. Furthermore, the machine learning can allow for a precise diagnosis of malignant lymphoma, based on features about an individual cell nucleus N, an individual cell C and a distribution state of cell C, digitized. Such a diagnosis can be combined with genome information, gene expression information, protein information or clinical information to perform the machine learning, thereby allowing for a more precise diagnosis of malignant lymphoma. The machine learning method is not particularly limited as long as it can be applied to the extractor 10, the digitizer 11 and the estimator 12, and any learning method such as supervised learning, unsupervized learning or reinforcement learning can be applied.

In Embodiment 1 described above, the information on malignant lymphoma, namely, the estimated information 5 includes the presence/absence, the class, the progress stage, and the like. However, the present disclosure is not limited thereto. Any one or at least two of the presence/absence, class and progress stage of malignant lymphoma may be estimated as the information on malignant lymphoma. The estimated information 5 may also include information representing prediction of prognoses (vital prognosis, functional prognosis and social prognosis) of malignant lymphoma.

### (Evaluation result 1)

The diagnosis device 1 according to the above embodiment was used to calculate features of cell nucleus forms (shapes (internal textures), colors, outer shapes) and the like of diffuse large-cell B cell lymphoma (hereinafter, referred to as DLBCL), follicular lymphoma (hereinafter, referred to as FL), reactive lymphoadenitis (hereinafter, referred to as Reactive), and the like, in the form of numerical information, and the effect due to feature digitization was confirmed by linear discriminant analysis.

Herein, 30 cell nucleus images of 450 × 450 pixels were defined as a data set, and evaluated. Each feature digitization was performed in the diagnosis device 1 with the following parameters representing 22 features.

### Parameters about shape (internal texture)

a: Area: area
b: Perimeter: outer perimeter
c: MajorRectLength: principal axis (longer axis) of rect structure (quadrangle enclosing cell nucleus)
d: MinorRectLength: accessory axis (shorter axis) of rect structure
e: ConvexRatio: expansion rate
f: MajorEllipLength: principal axis (longer axis) of ellipse approximation
g: MinorEllipLength: accessory axis (shorter axis) of ellipse approximation
h: Eccentricity: degree of eccentricity
i: Roundness: roundness

### Parameters about color

j: MeanGray: average value of brightness value
k: StdDevGray: standard deviation of brightness value
l: ModeGray: mode of brightness value, brightness value highest in frequency of appearance
m: MaxGray: maximum brightness value
o: Skewness: three-moment of brightness value
p: Kurtosis: fourth-moment of brightness value

### Parameters about outer shape

q: ConcaveCount: number of depressed regions in visible outline
r to v: First k PCA components of contour coordinates (k = 5): the top k = 5 (mutually perpendicular) deformation components suitable for expression of a variety of visible outline shapes, obtained by principle component analysis, namely, the first k = 5 components in arrangement of deformation components in the ordering of strength (magnification of dispersion of shapes along with the deformation components)

The contour coordinates mean those expressing visible outline shapes by vertex coordinates in approximation of a visible outline by a polygon. When respective coordinates of four vertexes in a quadrangle are, for example, (0,0), (1,0), (1,1), and (0,1), the quadrangle is expressed by 2 × 4 = 8 numbers (0,0,1,0,1,1,0,1) where the coordinates of the four vertexes are arranged. When a visible outline is approximated to a hectogon, the contour coordinates are expressed by a data row of 2 × 100 = 200 pieces of data.

When N visible outlines each expressed by 200 pieces of data are present, the average with respect to these visible outlines is determined by wholly adding 200 pieces of data with respect to each component and dividing the sum by N. This feature teaches which deformation component is strong relative to the average shape in a case where shapes of N visible outlines are varied. Principle component analysis can be applied to thereby provide a component to be scaled, for example, in a circular shape state kept with the gravity center of a cell nucleus as the center, and also a deformation component not only to be so scaled, but also to be scaled along with only one axis, as components.

Hereinafter, the description is made with the above parameters representing 22 features respectively designated as a to v.

The above 30 data sets were used to perform linear discriminant analysis with the above parameters a to v representing 22 features, and a first axis and a second axis, where DLBCL, FL and Reactive were able to be most favorably discriminated, were obtained. The first axis and the second axis respectively indicate values of a first discrimination function and a second discrimination function obtained by linear discriminant analysis.

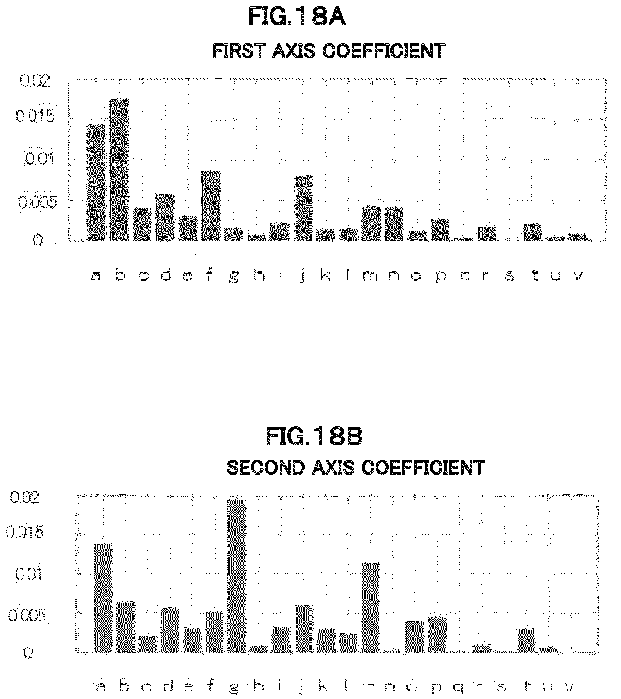

Graphs illustrated in FIG. 18A and FIG. 18B illustrate the weights of respective features about the first axis and the second axis obtained by linear discriminant analysis, namely, a first axis coefficient of each of parameters a to v, corresponding to the first axis, and a second axis coefficient of each of parameters a to v, corresponding to the second axis. It is indicated that, as the values of coefficients are larger, the weight of each of features a to v relative to the first discrimination function is larger. As illustrated in FIG. 18A and FIG. 18B, the largest weight to the classification in the first axis is observed in "b: outer perimeter", and the highest degree of contribution to the classification in the second axis is observed in "g: accessory axis (shorter axis) of ellipse approximation".

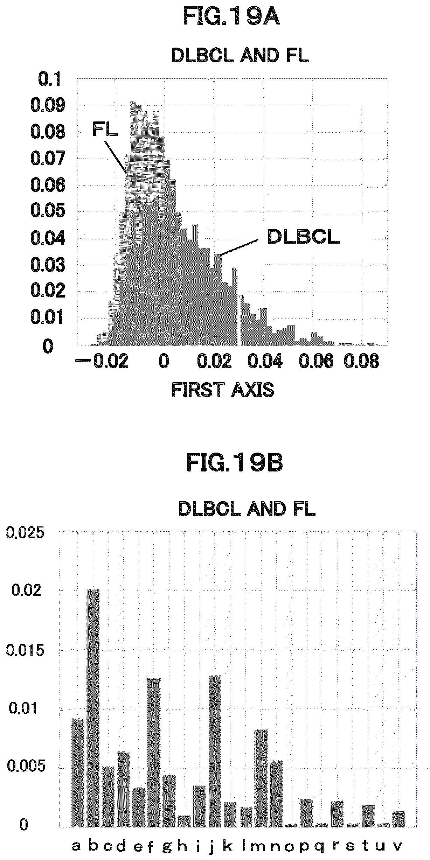

FIG. 19A illustrates distribution states (probability densities) of features of a cell nucleus of DLBCL and a cell nucleus of FL in a first axis direction, in the case of linear discriminant analysis performed with the above parameters representing 22 features a to v. As illustrated in FIG. 19A, it has been revealed that the respective features of cell nuclei of DLBCL and FL in the first axis direction follow different distributions. FIG. 19B illustrates the weight of each feature about the first axis obtained by linear discriminant analysis, namely, the first axis coefficient. In this case, the largest weight is observed in "b: outer perimeter".

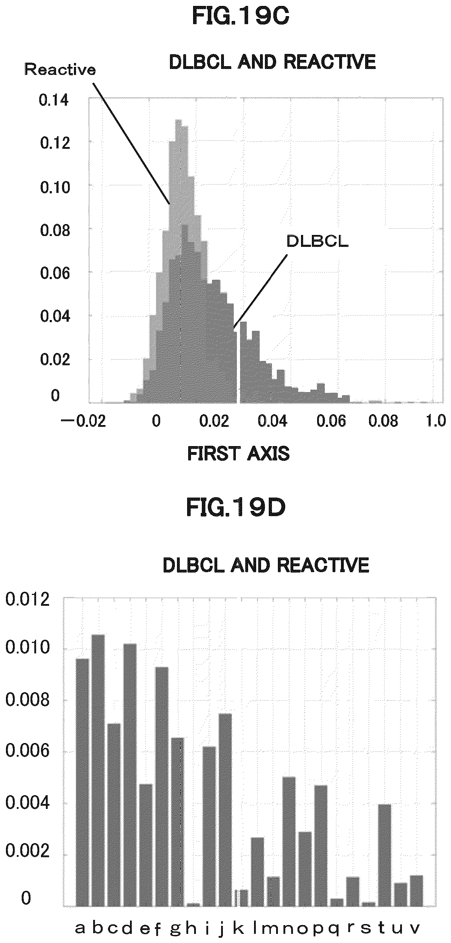

FIG. 19C illustrates distribution states (probability densities) of features of a cell nucleus of DLBCL and a cell nucleus of Reactive in a first axis direction, in the case of linear discriminant analysis performed with the above parameters representing 22 features a to v. As illustrated in FIG. 19C, it has been revealed that the respective features of cell nuclei of DLBCL and Reactive in the first axis direction follow different distributions. FIG. 19D illustrates the weight of each feature about the first axis obtained by linear discriminant analysis, namely, the first axis coefficient. In this case, the largest weight is observed in "b: outer perimeter", but the weights observed in the coefficients d, a, f, and the like are also larger.

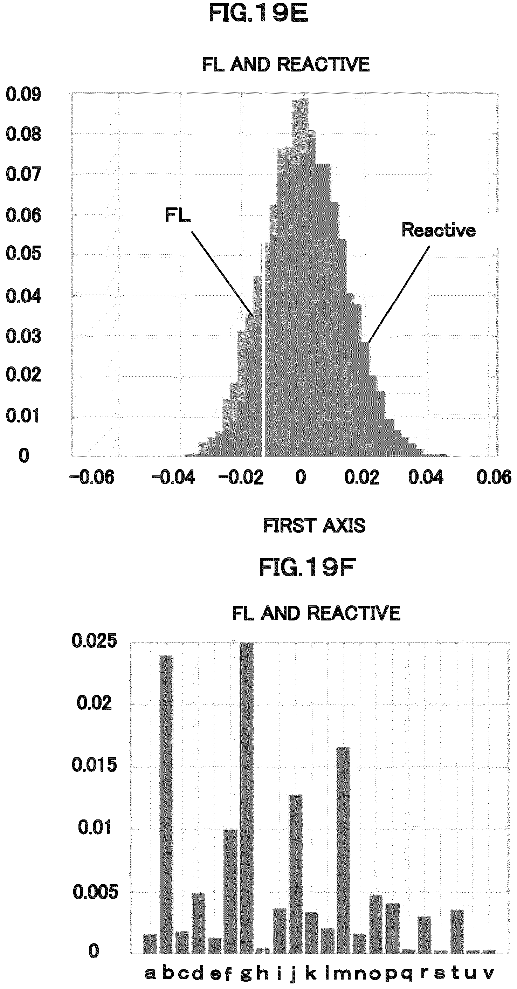

FIG. 19E illustrates distribution states of features (probability densities) of a cell nucleus of FL and a cell nucleus of Reactive in a first axis direction, in the case of linear discriminant analysis performed with the above parameters representing 22 features a to v. As illustrated in FIG. 19E, it has been revealed that the respective features of cell nuclei of FL and Reactive in the first axis direction follow different distributions. FIG. 19F illustrates the weight of each feature about the first axis obtained by linear discriminant analysis, namely, the first axis coefficient. In this case, the largest weight is observed in "g: accessory axis (shorter axis) of ellipse approximation".

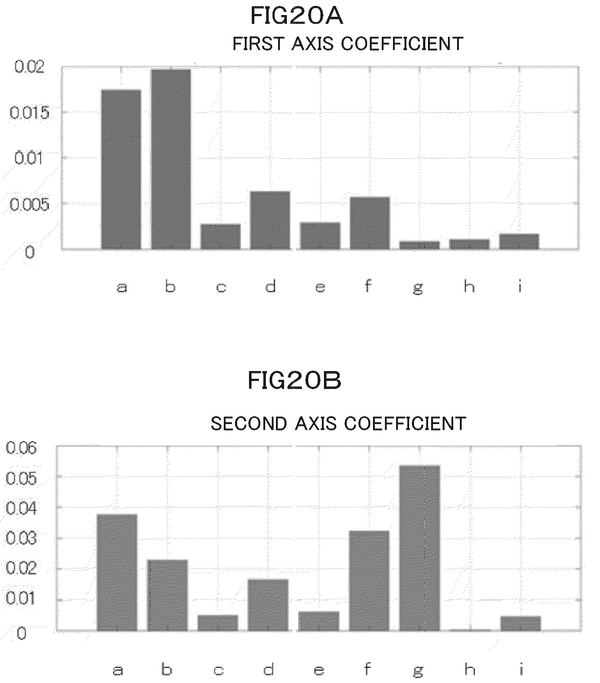

The nine parameters a to i, about a shape (internal texture), among the above 22 features, were used to perform linear discriminant analysis of DLBCL, FL and Reactive, and a first axis and a second axis, where DLBCL, FL and Reactive were able to be most favorably discriminated, were obtained. Graphs illustrated in FIG. 20A and FIG. 20B illustrates the weights of respective features about the first axis and the second axis obtained by linear discriminant analysis, namely, the first axis coefficient of each of parameters a to i, corresponding to the first axis, and the second axis coefficient of each of parameters a to i, corresponding to the second axis. As illustrated in FIG. 20A and FIG. 20B, the highest degree of contribution to the classification in the first axis is observed in "b: outer perimeter", and the highest degree of contribution to the classification in the second axis is observed in "g: accessory axis (shorter axis) of ellipse approximation".

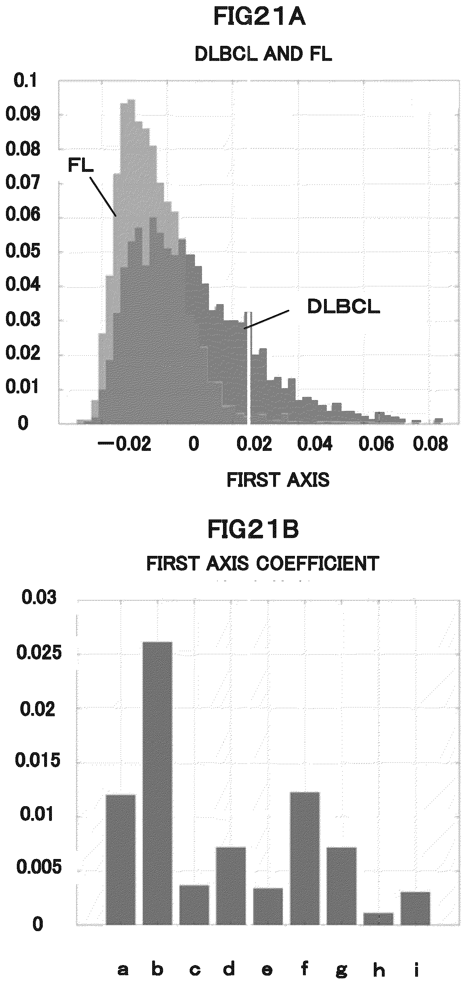

FIG. 21A illustrates distribution states of features (probability densities) of a cell nucleus of DLBCL and a cell nucleus of FL in a first axis direction, in the case of linear discriminant analysis performed with parameters a to i representing features about the shape of a cell nucleus. As illustrated in FIG. 21A, it has been revealed that the respective features of cell nuclei of DLBCL and FL in the first axis direction follow different distributions. FIG. 21B illustrates the weight of each feature about the first axis obtained by linear discriminant analysis, namely, the first axis coefficient. In this case, the largest weight is observed in "b: outer perimeter".

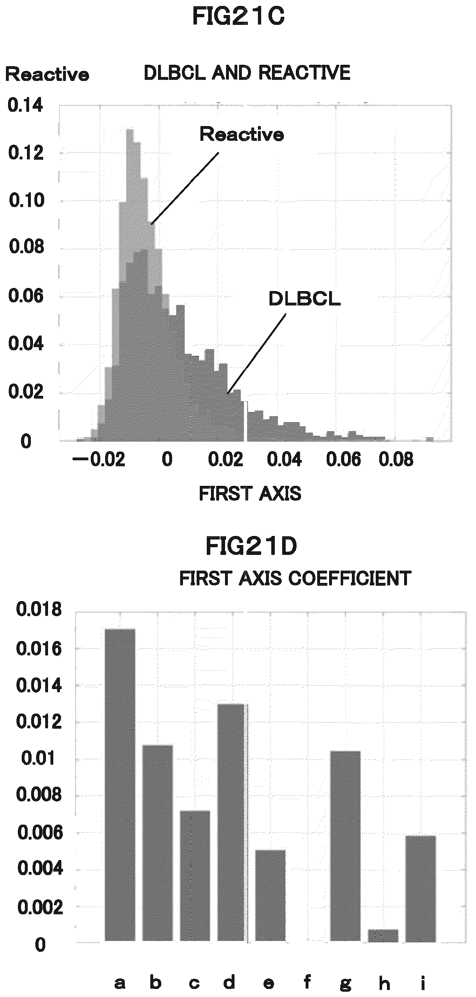

FIG. 21C illustrates distribution states of features (probability densities) of a cell nucleus of DLBCL and a cell nucleus of Reactive in a first axis direction, in the case of linear discriminant analysis performed with parameters a to i representing features about the shape of a cell nucleus. As illustrated in FIG. 21C, it has been revealed that the respective features of cell nuclei of DLBCL and Reactive in the first axis direction follow different distributions. FIG. 21D illustrates the weight of each feature about the first axis obtained by linear discriminant analysis, namely, the first axis coefficient. In this case, the largest weight is observed in "a: area".

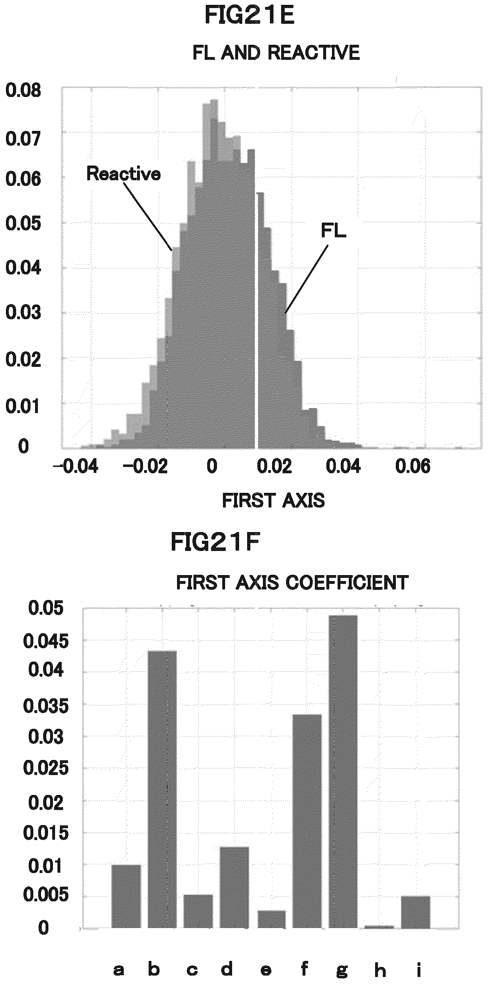

FIG. 21E illustrates distribution states of features (probability densities) of a cell nucleus of FL and a cell nucleus of Reactive in a first axis direction, in the case of linear discriminant analysis performed with parameters a to i representing features about the shape of a cell nucleus. As illustrated in FIG. 21E, it has been revealed that the respective features of cell nuclei of FL and Reactive in the first axis direction follow different distributions. FIG. 21F illustrates the weight of each feature about the first axis obtained by linear discriminant analysis, namely, the first axis coefficient. In this case, the largest weight is observed in "g: accessory axis (shorter axis) of ellipse approximation".

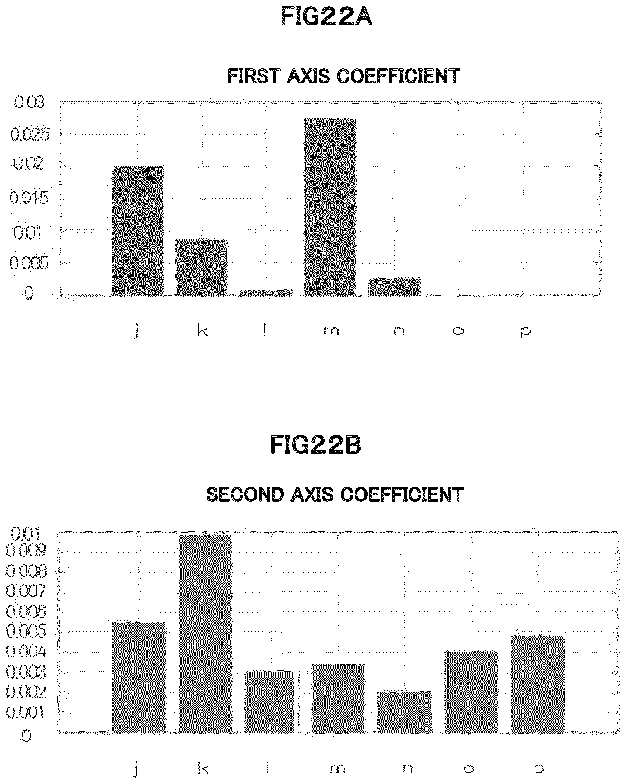

The above data sets were used and the seven parameters j to p, about a color, among the above 22 features, were adopted, and a first axis and a second axis, where DLBCL, FL and Reactive were able to be most favorably discriminated, were obtained. The first axis and the second axis respectively indicate values of a first discrimination function and a second discrimination function obtained by linear discriminant analysis. Graphs illustrated in FIG. 22A and FIG. 22B illustrate the weights of respective features about the first axis and the second axis obtained by linear discriminant analysis, namely, the first axis coefficient of each of parameters j to p, corresponding to the first axis, and the second axis coefficient of each of parameters j to p, corresponding to the second axis. As illustrated in FIG. 22A and FIG. 22B, the highest degree of contribution to the classification in the first axis is observed in "m: maximum brightness value", and the highest degree of contribution to the classification in the second axis is observed in "k: standard deviation of brightness value".

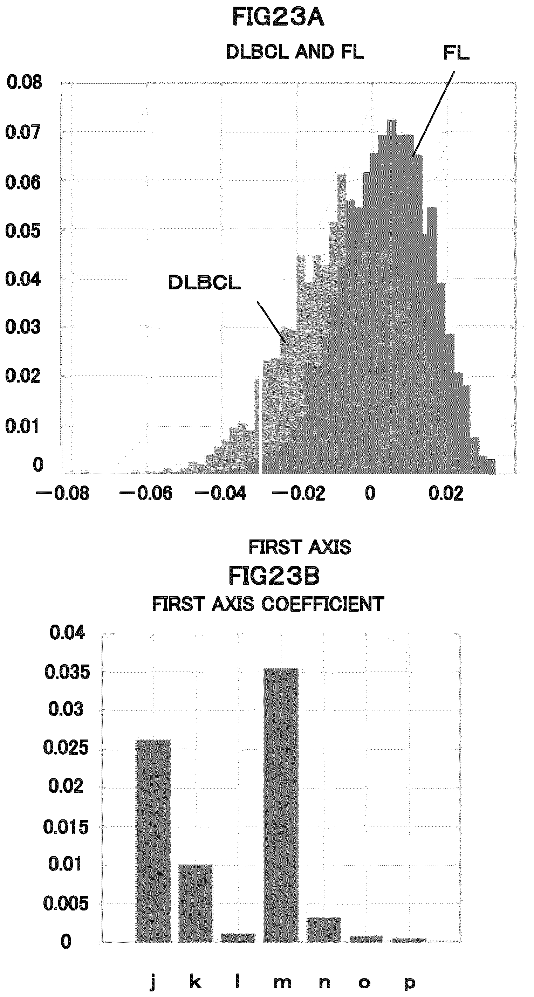

FIG. 23A illustrates distribution states of features (probability densities) of a cell nucleus of DLBCL and a cell nucleus of FL in a first axis direction, in the case of linear discriminant analysis performed with parameters j to p representing features about the color of a cell nucleus. As illustrated in FIG. 23A, it has been revealed that the respective features of cell nuclei of DLBCL and FL in the first axis direction follow different distributions. FIG. 23B illustrates the weight of each feature about the first axis obtained by linear discriminant analysis, namely, the first axis coefficient. In this case, the largest weight is observed in "m: maximum brightness value".

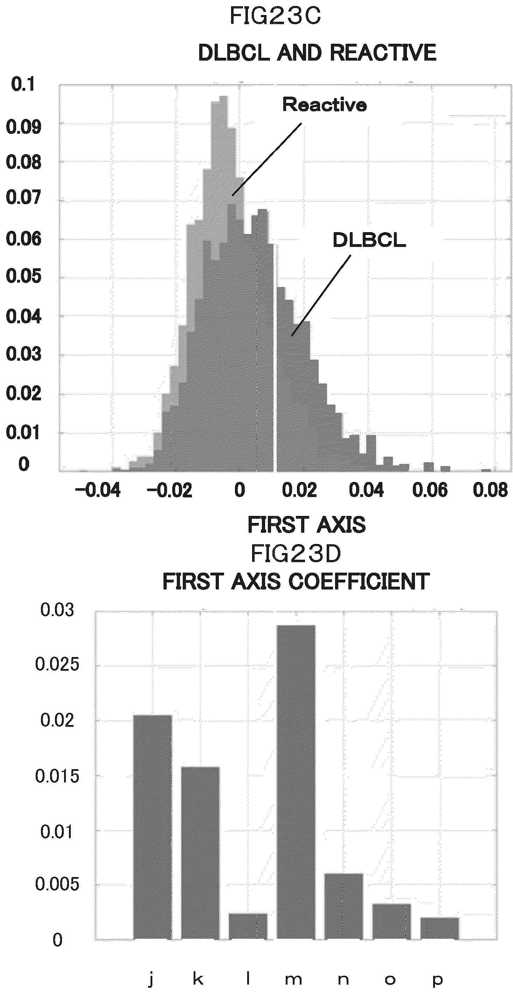

FIG. 23C illustrates distribution states of features (probability densities) of a cell nucleus of DLBCL and a cell nucleus of Reactive in a first axis direction, in the case of linear discriminant analysis performed with parameters j to p representing features about the color of a cell nucleus. As illustrated in FIG. 23C, it has been revealed that the respective features of cell nuclei of DLBCL and Reactive in the first axis direction follow different distributions. FIG. 23D illustrates the weight of each feature about the first axis obtained by linear discriminant analysis, namely, the first axis coefficient. In this case, the largest weight is observed in "m: maximum brightness value".

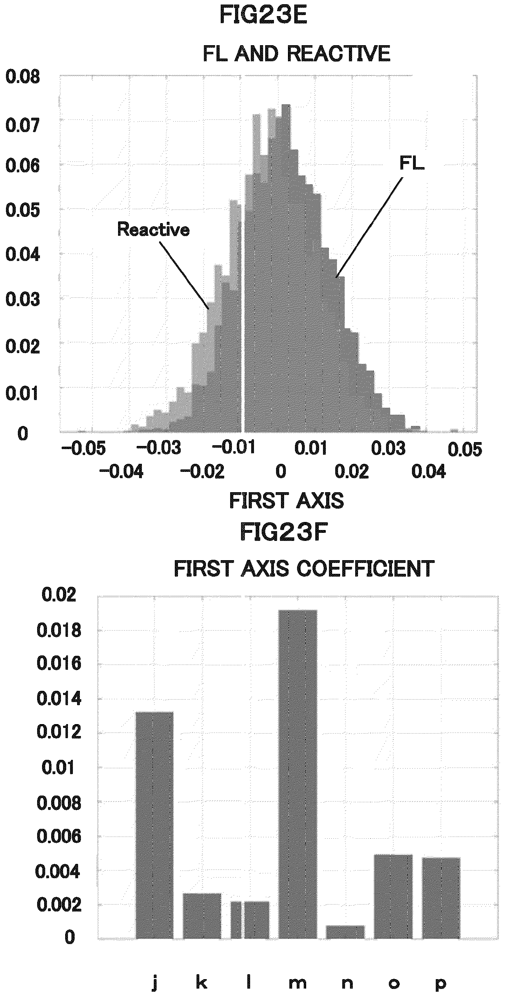

FIG. 23E illustrates distribution states of features (probability densities) of a cell nucleus of FL and a cell nucleus of Reactive in a first axis direction, in the case of linear discriminant analysis performed with parameters j to p representing features about the color of a cell nucleus. As illustrated in FIG. 23E, it has been revealed that the respective features of cell nuclei of FL and Reactive in the first axis direction follow different distributions. FIG. 23F illustrates the weight of each feature about the first axis obtained by linear discriminant analysis, namely, the first axis coefficient. In this case, the largest weight is observed in "m: maximum brightness value".

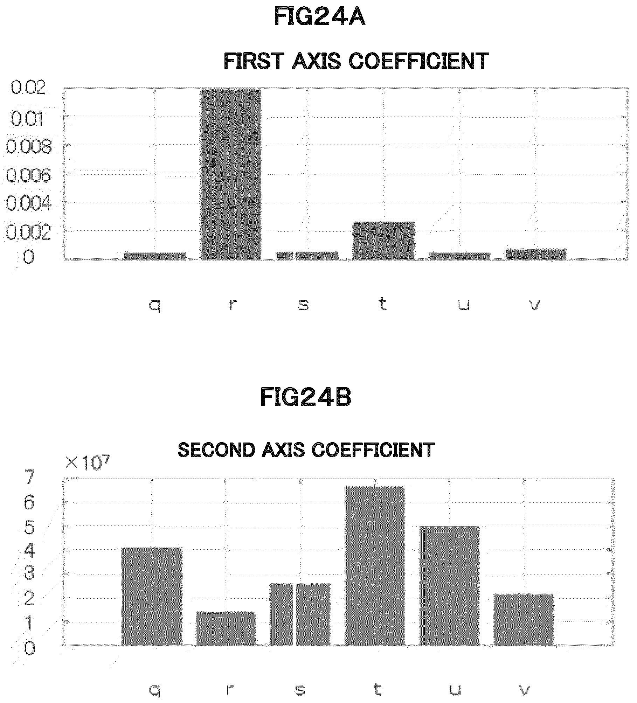

The parameters q to v representing six features about the outer shape of a cell nucleus were used to perform linear discriminant analysis, and a first axis and a second axis, where DLBCL, FL and Reactive were able to be most favorably discriminated, were obtained. Each graph represented in FIG. 24A and FIG. 24B illustrates the weight of each feature about the first axis obtained by linear discriminant analysis, namely, the first axis coefficient. As illustrated in FIG. 24A and FIG. 24B, the highest degree of contribution to the classification in the first axis is observed in "r: the top first (mutually perpendicular) deformation component suitable for expression of a variety of visible outline shapes, obtained in principle component analysis", and the highest degree of contribution to the classification in the second axis is observed in "t: the third (mutually perpendicular) deformation component suitable for expression of a variety of visible outline shapes, obtained in principle component analysis".

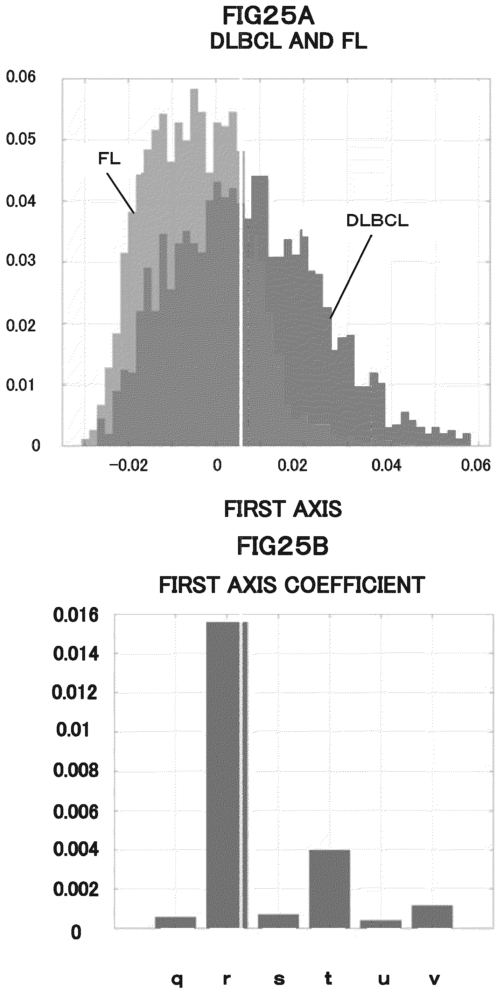

FIG. 25A illustrates distribution states of features (probability densities) of a cell nucleus of DLBCL and a cell nucleus of FL in a first axis direction, in the case of linear discriminant analysis performed with parameters q to v representing features about the outer shape of a cell nucleus. As illustrated in FIG. 25A, it has been revealed that the respective features of cell nuclei of DLBCL and FL in the first axis direction follow different distributions. FIG. 25B illustrates the weight of each feature about the first axis obtained by linear discriminant analysis, namely, the first axis coefficient. In this case, the largest weight is observed in "r: the top first (mutually perpendicular) deformation component suitable for expression of a variety of visible outline shapes, obtained in principle component analysis".

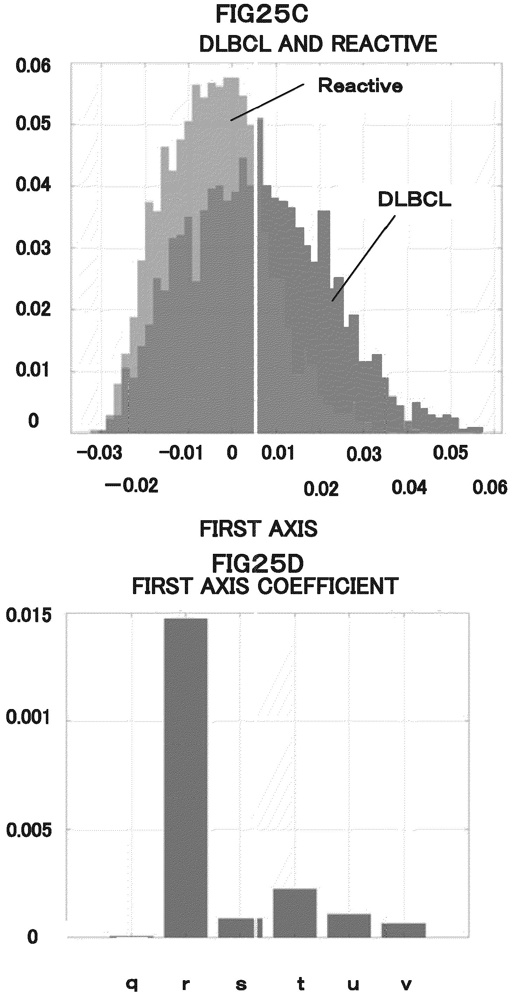

FIG. 25C illustrates distribution states of features (probability densities) of a cell nucleus of DLBCL and a cell nucleus of Reactive in a first axis direction, in the case of linear discriminant analysis performed with parameters q to v representing features about the outer shape of a cell nucleus. As illustrated in FIG. 25C, it has been revealed that the respective features of cell nuclei of DLBCL and Reactive in the first axis direction follow different distributions. FIG. 25D illustrates the weight of each feature about the first axis obtained by linear discriminant analysis, namely, the first axis coefficient. In this case, the largest weight is observed in "r: the top first (mutually perpendicular) deformation component suitable for expression of a variety of visible outline shapes, obtained in principle component analysis".

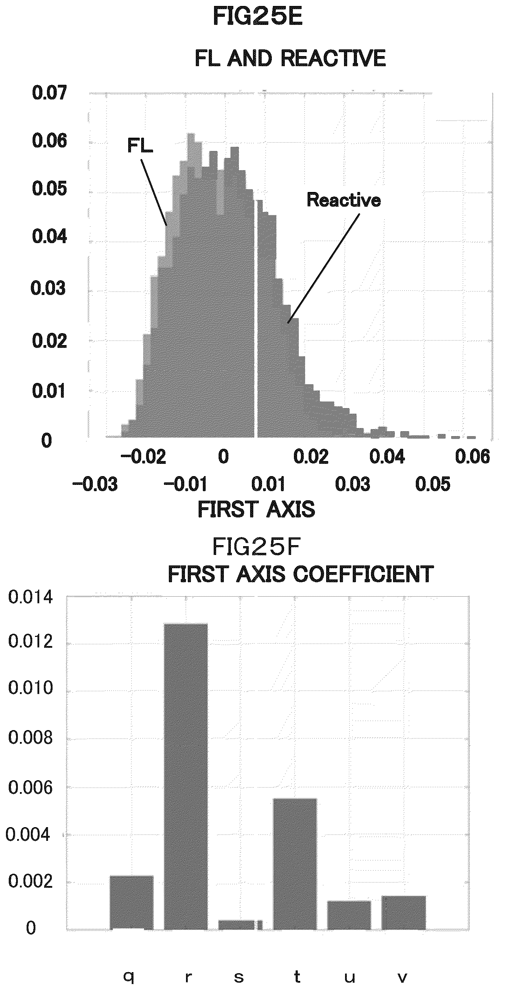

FIG. 25E illustrates distribution states of features (probability densities) of a cell nucleus of FL and a cell nucleus of Reactive in a first axis direction, in the case of linear discriminant analysis performed with parameters q to v representing features about the outer shape of a cell nucleus. As illustrated in FIG. 25E, it has been revealed that the respective features of cell nuclei of FL and Reactive in the first axis direction follow different distributions. FIG. 25F illustrates the weight of each feature about the first axis obtained by linear discriminant analysis, namely, the first axis coefficient. In this case, the largest weight is observed in "r: the top first (mutually perpendicular) deformation component suitable for expression of a variety of visible outline shapes, obtained in principle component analysis".

### (Evaluation result 2)

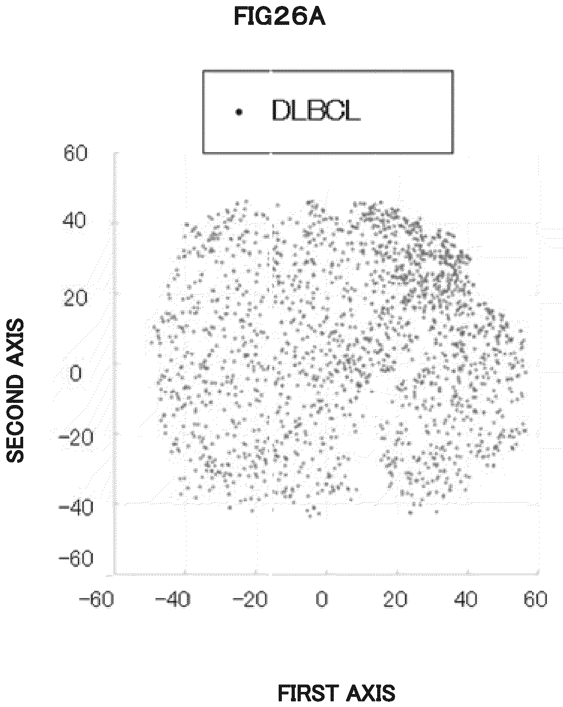

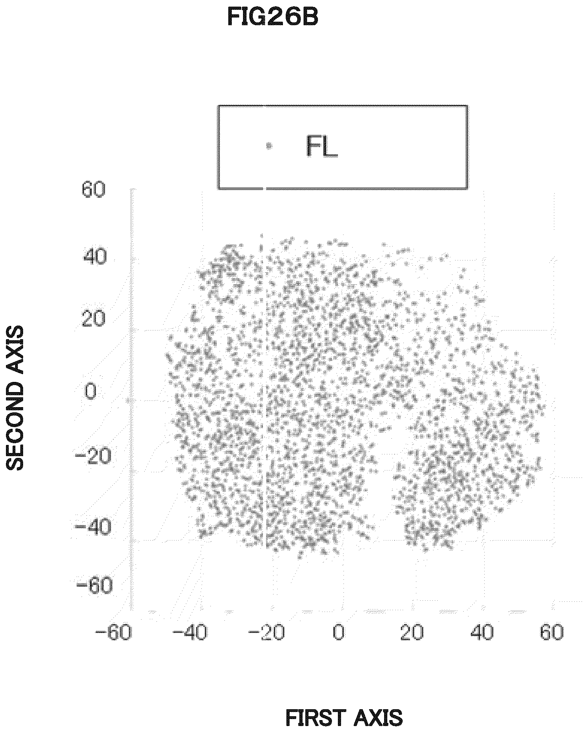

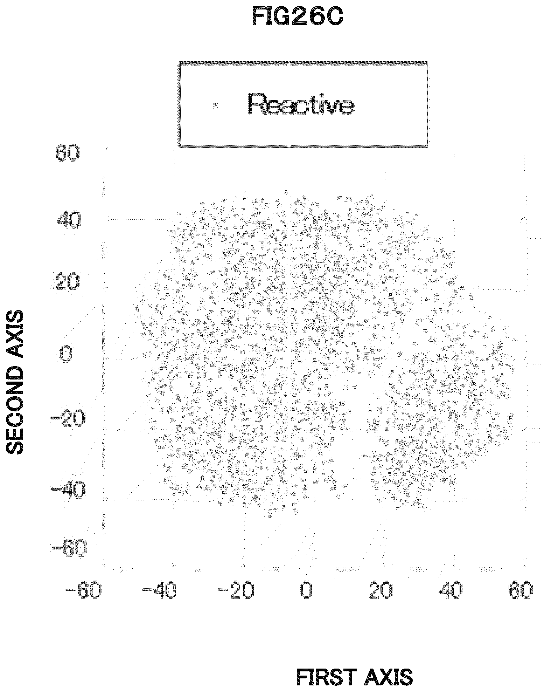

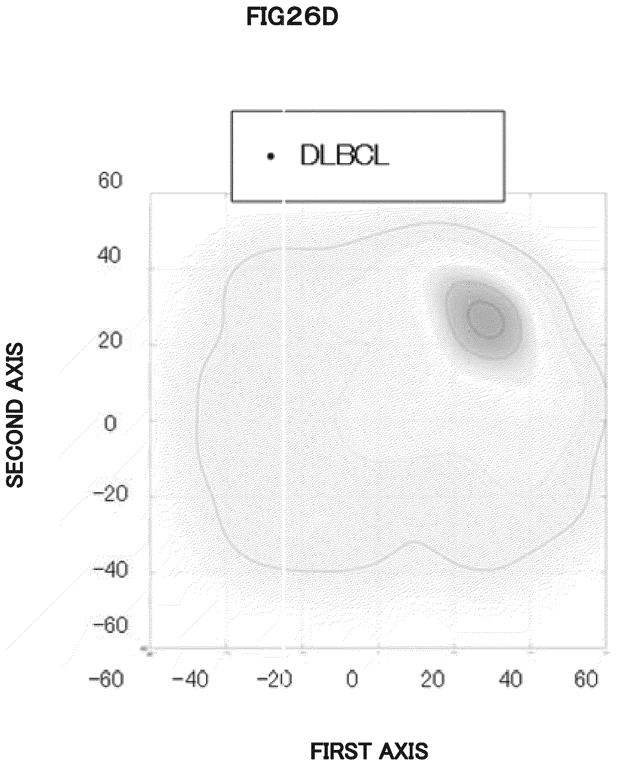

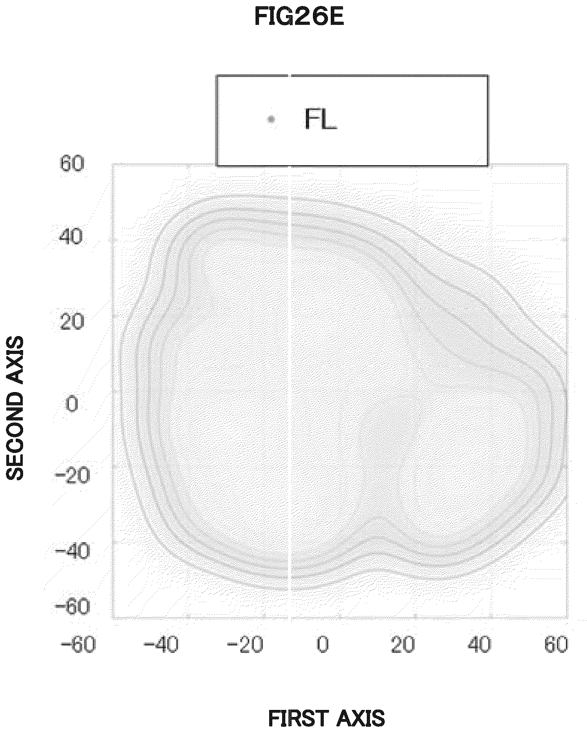

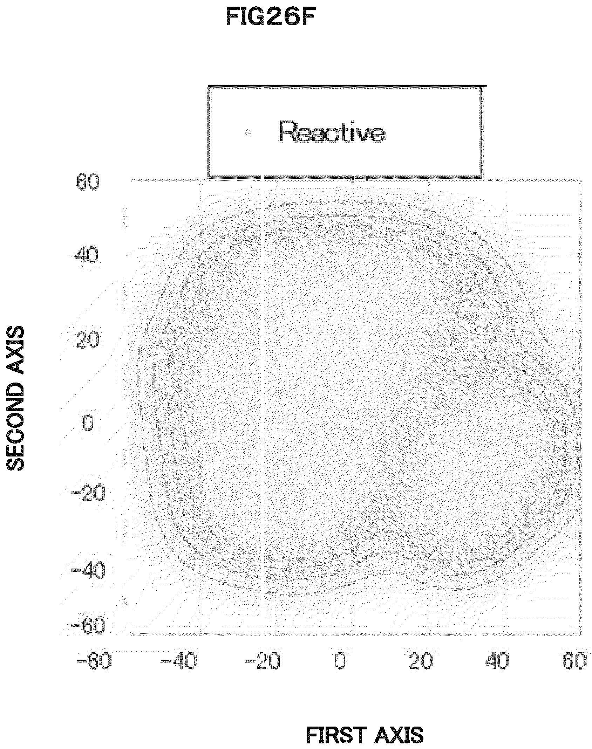

The diagnosis device 1 according to the above embodiment was used to calculate cell nucleus forms of DLBCL, FL and Reactive, as numerical information, and TSNE (t-Distributed Stochastic Neighbor Embedding) analysis was performed for reduction to two dimension (first axis, second axis). FIG. 26A represents a distribution of a feature of a cell nucleus of DLBCL, FIG. 26B represents a distribution of a feature of a cell nucleus of FL, and FIG. 26C represents a distribution of a feature of a cell nucleus of Reactive. FIG. 26D represents a probability density of a feature of a cell nucleus of DLBCL, FIG. 26E represents a probability density of a feature of a cell nucleus of FL, and FIG. 26F represents a probability density of a feature of a cell nucleus of Reactive. As can be seen from these drawings, the presence of cell nucleus forms specific for DLBCL and, FL and, Reactive can be visualized.

The hardware configuration and software configuration of the diagnosis device 1 are thus illustrative, and can be arbitrarily changed and modified.

A section mainly responsible for performing processing of the diagnosis device 1 configured from the CPU 21, the main storage 22, the external storage 23, the operating section 24, the display 25, the communication interface 26, the internal bus 28, and the like is not limited to any dedicated system, and such processing can be realized with a usual computer system. For example, the diagnosis device 1 may be constituted so that the processing is executed by storing a computer program for executing the operation, in a recording medium (flexible disc, CD-ROM, DVD-ROM, or the like) readable by a computer, distributing the computer program, and installing the computer program in the computer. The diagnosis device 1 may also be constituted so that the computer program is stored in a storage device contained in a server device on a communication network such as internet and the computer program is downloaded by a usual computer system.

In a case where the function of the diagnosis device 1 is realized by assignation to OS (operating system) and an application program, or cooperation of OS and an application program, only an application program section may be stored in a recording medium or a storage device.

It is also possible to superpose a computer program with carrier wave and distribute the program via a communication network. For example, a computer program may be put up on a message board (BBS, Bulletin Board System) on a communication network, and the computer program may be distributed via a network. The diagnosis device may also be constituted so that the processing can be executed by activating the computer program and executing it under control with OS in the same manner as in other application program.

The foregoing describes some example embodiments for explanatory purposes. Although the foregoing discussion has presented specific embodiments, persons skilled in the art will recognize that changes may be made in form and detail without departing from the scope of the invention. Accordingly, the specification and drawings are to be regarded in an illustrative rather than a restrictive sense. This detailed description, therefore, is not to be taken in a limiting sense, and the scope of the invention is defined only by the included claims.

This application claims the benefit of Japanese Patent Application No. 2021-131711, filed on August 12, 2021.

### Industrial Applicability

The present disclosure can be applied to a diagnosis of malignant lymphoma.

### Reference Signs List

- 1: Diagnosis device

- 2, 2a: Pathological image
- 2A: Sample image
- 3: Extraction information
- 3A, 3B, 3C: Sample image
- 3a: Cell nucleus extraction information
- 3b: Cell extraction information
- 3c: Distribution state extraction information
- 4, 4A: Numerical information
- 4a, 4a': Cell nucleus numerical information
- 4b, 4b': Cell numerical information
- 4c, 4c': Distribution state numerical information
- 5, 5A: Estimated information
- 5a,: 5a'Presence/absence information
- 5b, 5b': Class information
- 5c,: 5c'Progress stage information
- 5d: Genome information
- 5e: Gene expression information
- 5f: Protein information
- 5g: Clinical information
- 6: Additional information
- 6a: Genome information
- 6b: Gene expression information
- 6c: Protein information
- 6d: Clinical information
- 10: Extractor
- 11: Digitizer
- 12: Estimator
- 21: CPU
- 22: Main storage
- 23: External storage
- 24: Operating section
- 25: Display
- 26: Communication interface
- 28: Internal bus
- 29: Diagnosis program
- 30: Machine learning program
- 31, 31A, 31B: Sample data
- C: Cell
- G1: First image
- G2: Second image
- G1A: First sample image
- G2A: Second sample image
- N: Cell nucleus
- P: Aggregate pattern

## Claims

1. A diagnosis device comprising:
an extractor that extracts an individual cell nucleus, an individual cell, and a distribution state of cell from a pathological image in which a plurality of cells including a lymphoid cell is photographed; and
a digitizer that digitizes a feature of an individual cell nucleus, a feature of an individual cell, and a feature of a distribution state of cell based on the individual cell nucleus, the individual cell, and the distribution state of cell extracted by the extractor, further comprising:
an estimator that estimates information on malignant lymphoma based on a feature of an individual cell nucleus, a feature of an individual cell, and a feature of a distribution state of cell, digitized by the digitizer,
wherein the estimator
performs machine learning based on sample data representing a relationship between a feature of an individual cell nucleus, a feature of an individual cell and a feature of a distribution state of cell, and information on malignant lymphoma, and
estimates information on malignant lymphoma from a feature of an individual cell nucleus, a feature of an individual cell and a feature of a distribution state of cell, digitized by the digitizer, based on a result of the machine learning, and
wherein
the estimator estimates information on malignant lymphoma based on not only an individual cell nucleus, a feature of an individual cell and a feature of a distribution state of cell, digitized by the digitizer, but also at least one additional information of genome information, gene expression information, protein information or clinical information correlated with malignant lymphoma.

2. The diagnosis device according to claim 1, wherein
the extractor
reads a pathological image obtained by taking a range in which a feature of a distribution state of cell is recognizable, at a resolution at which features of an individual cell nucleus and an individual cell are recognizable, and
extracts an individual cell nucleus, an individual cell, and a distribution state of cell from the pathological image read.

3. The diagnosis device according to claim 2, wherein
the extractor
performs machine learning based on sample data representing a relationship between a sample image as a sample of the pathological image, and an individual cell nucleus, an individual cell and a distribution state of cell manually extracted from the sample image, and
extracts an individual cell nucleus, an individual cell, and a distribution state of cell from the pathological image, based on a result of the machine learning.

4. The diagnosis device according to claim 1, wherein
the extractor not only extracts an individual cell, an individual cell nucleus and a distribution state of cell from a first image having a resolution at which an individual cell nucleus and an individual cell taken from a living organism are recognizable, but also extracts a distribution state of cell from a second image containing a range in which a feature of a distribution state of cell taken from the living organism is recognizable.

5. The diagnosis device according to claim 4, wherein
the extractor
not only performs machine learning based on sample data representing a relationship between a first sample image corresponding to the first image, and a cell and a cell nucleus manually extracted from the first sample image, but also performs machine learning based on sample data representing a relationship between a second sample image corresponding to the second image, and a distribution state of cell manually extracted from the second sample image, and
not only extracts an individual cell and an individual cell nucleus from the first image, but also extracts a distribution state of cell from the second image, based on a result of the machine learning.

6. The diagnosis device according to any one of claims 1 to 4, wherein
the digitizer digitizes a feature of an individual cell nucleus, a feature of an individual cell and a feature of a distribution state of cell correlated with information on malignant lymphoma, based on the individual cell nucleus, the individual cell, and the distribution state of cell extracted by the extractor.

7. The diagnosis device according to claim 6, wherein
the digitizer
performs machine learning based on sample data representing a relationship between a feature of an individual cell nucleus, a feature of an individual cell and a feature of a distribution state of cell, and information on malignant lymphoma, and
narrows a feature of an individual cell nucleus, a feature of an individual cell and a feature of a distribution state of cell, down to a feature to be digitized, based on a result of the machine learning.

8. The diagnosis device according to any one of claims 1 to 7, wherein information on malignant lymphoma includes at least one of information representing presence/absence, a class or a progress stage of malignant lymphoma, or genome information, gene expression information, protein information or clinical information correlated with malignant lymphoma.

9. The diagnosis device according to any one of claims 1 to 8, wherein
the estimator
performs machine learning based on sample data representing a relationship between a feature of an individual cell nucleus, a feature of an individual cell, a feature of a distribution state of cell and the additional information, and information on malignant lymphoma, and
estimates information on malignant lymphoma based on a result of the machine learning.

10. The diagnosis device according to any one of claims 1 to 9, wherein
the cell includes a vascular endothelial cell, a mesenchymal cell including a fibroblast cell, a blood cell other than a lymphoid cell, including histiocyte, an epithelial cell, or a neural cell.

11. An information acquisition method executed by an information-processing device, wherein the processing is executed by a diagnosis device according to any one of claims 1 to 10, the method comprising:
extracting an individual cell nucleus, an individual cell, and a distribution state of cell from a pathological image in which a plurality of cells including a lymphoid cell is photographed; and
digitizing a feature of an individual cell nucleus, a feature of an individual cell, and a feature of a distribution state of cell based on the extracted individual cell nucleus, the extracted individual cell, and the extracted distribution state of cell.

12. A program causing a computer to serve as a diagnosis device according to any one of claims 1 to 10.

## Patentansprüche

1. Diagnosevorrichtung, umfassend:
eine Extraktionseinrichtung, die einen einzelnen Zellkern, eine einzelne Zelle und einen Verteilungszustand einer Zelle aus einem pathologischen Bild extrahiert, in dem eine Mehrzahl von Zellen, einschließlich eine Lymphoidzelle, fotografiert ist; und
eine Digitalisierungseinrichtung, die ein Merkmal eines einzelnen Zellkerns, ein Merkmal einer einzelnen Zelle und ein Merkmal eines Verteilungszustands einer Zelle auf der Basis des einzelnen Zellkerns, der einzelnen Zelle und des Verteilungszustands der Zelle, die durch die Extraktionseinrichtung extrahiert worden sind, digitalisiert,
ferner umfassend:
eine Abschätzungseinrichtung, die Informationen bezüglich eines malignen Lymphoms auf der Basis eines Merkmals eines einzelnen Zellkerns, eines Merkmals einer einzelnen Zelle und eines Merkmals eines Verteilungszustands einer Zelle, die durch die Digitalisierungseinrichtung digitalisiert worden sind, abschätzt,
wobei die Abschätzungseinrichtung
ein maschinelles Lernen auf der Basis von Probendaten durchführt, die eine Beziehung zwischen einem Merkmal eines einzelnen Zellkerns, einem Merkmal einer einzelnen Zelle und einem Merkmal eines Verteilungszustands einer Zelle und Informationen über ein malignes Lymphom darstellen, und
Informationen bezüglich eines malignen Lymphoms aus einem Merkmal eines einzelnen Zellkerns, einem Merkmal einer einzelnen Zelle und einem Merkmal eines Verteilungszustands einer Zelle, die durch die Digitalisierungseinrichtung digitalisiert worden sind, auf der Basis eines Ergebnisses des maschinellen Lernens abschätzt, und
wobei
die Abschätzungseinrichtung Informationen bezüglich eines malignen Lymphoms nicht nur auf der Basis eines einzelnen Zellkerns, eines Merkmals einer einzelnen Zelle und eines Merkmals eines Verteilungszustands einer Zelle, die durch die Digitalisierungseinrichtung digitalisiert worden sind, sondern mindestens auch einer zusätzlichen Information von Genominformationen, Genexpressionsinformationen, Proteininformationen oder klinischen Informationen, die mit einem malignen Lymphom korrelieren, abschätzt.

2. Diagnosevorrichtung nach Anspruch 1, wobei
die Extraktionseinrichtung
ein pathologisches Bild, das durch Verwenden eines Bereichs, in dem ein Merkmal eines Verteilungszustands einer Zelle erkennbar ist, erhalten wird, bei einer Auflösung ausliest, bei der Merkmale eines einzelnen Zellkerns und einer einzelnen Zelle erkennbar sind, und
einen einzelnen Zellkern, eine einzelne Zelle und einen Verteilungszustand einer Zelle von dem ausgelesenen pathologischen Bild extrahiert.

3. Diagnosevorrichtung nach Anspruch 2, wobei
die Extraktionseinrichtung
ein maschinelles Lernen auf der Basis von Probendaten, die eine Beziehung zwischen einem Probenbild als eine Probe des pathologischen Bilds, und einem einzelnen Zellkern, einer einzelnen Zelle und einem Verteilungszustand einer Zelle, die manuell von dem Probenbild extrahiert worden sind, durchführt, und
einen einzelnen Zellkern, eine einzelne Zelle und einen Verteilungszustand einer Zelle von dem pathologischen Bild auf der Basis eines Ergebnisses des maschinellen Lernens extrahiert.

4. Diagnosevorrichtung nach Anspruch 1, wobei
die Extraktionseinrichtung nicht nur eine einzelne Zelle, einen einzelnen Zellkern und einen Verteilungszustand einer Zelle von einem ersten Bild extrahiert, das eine Auflösung aufweist, bei der ein einzelner Zellkern und eine einzelne Zelle, die von einem lebenden Organismus entnommen worden sind, erkennbar sind, sondern auch einen Verteilungszustand einer Zelle von einem zweiten Bild extrahiert, das einen Bereich enthält, in dem ein Merkmal eines Verteilungszustands einer Zelle, die von dem lebenden Organismus entnommen worden ist, erkennbar ist.

5. Diagnosevorrichtung nach Anspruch 4, wobei
die Extraktionseinrichtung
nicht nur ein maschinelles Lernen auf der Basis von Probendaten durchführt, die eine Beziehung zwischen einem ersten Probenbild, das dem ersten Bild entspricht, und einer Zelle und einem Zellkern, die manuell von dem ersten Probenbild extrahiert worden sind, darstellen, sondern auch ein maschinelles Lernen auf der Basis von Probendaten durchführt, die eine Beziehung zwischen einem zweiten Probenbild, das dem zweiten Bild entspricht, und einem Verteilungszustand einer Zelle, der manuell von dem zweiten Probenbild extrahiert worden ist, darstellen, und
nicht nur eine einzelne Zelle und einen einzelnen Zellkern von dem ersten Bild extrahiert, sondern auch einen Verteilungszustand einer Zelle von dem zweiten Bild auf der Basis eines Ergebnisses des maschinellen Lernens extrahiert.

6. Diagnosevorrichtung nach einem der Ansprüche 1 bis 4, wobei
die Digitalisierungseinrichtung ein Merkmal eines einzelnen Zellkerns, ein Merkmal einer einzelnen Zelle und ein Merkmal eines Verteilungszustands einer Zelle, die mit Informationen bezüglich eines malignen Lymphoms korrelieren, auf der Basis des einzelnen Zellkerns, der einzelnen Zelle und des Verteilungszustands einer Zelle, die durch die Extraktionseinrichtung extrahiert worden sind, digitalisiert.

7. Diagnosevorrichtung nach Anspruch 6, wobei
die Digitalisierungseinrichtung
ein maschinelles Lernen auf der Basis von Probendaten durchführt, die eine Beziehung zwischen einem Merkmal eines einzelnen Zellkerns, einem Merkmal einer einzelnen Zelle und einem Merkmal eines Verteilungszustands einer Zelle und Informationen bezüglich eines malignen Lymphoms darstellen, und
ein Merkmal eines einzelnen Zellkerns, ein Merkmal einer einzelnen Zelle und ein Merkmal eines Verteilungszustands einer Zelle auf der Basis eines Ergebnisses des maschinellen Lernens zu einem zu digitalisierenden Merkmal verengt.

8. Diagnosevorrichtung nach einem der Ansprüche 1 bis 7, wobei
Informationen bezüglich eines malignen Lymphoms mindestens eines von Informationen, die das Vorliegen/Fehlen, eine Klasse oder ein Fortschrittstadium eines malignen Lymphoms darstellen, oder Genominformationen, Genexpressionsinformationen, Proteininformationen oder klinische Informationen, die mit einem malignen Lymphom korrelieren, umfassen.

9. Diagnosevorrichtung nach einem der Ansprüche 1 bis 8, wobei die Abschätzungseinrichtung
ein maschinelles Lernen auf der Basis von Probendaten durchführt, die eine Beziehung zwischen einem Merkmal eines einzelnen Zellkerns, einem Merkmal einer einzelnen Zelle, einem Merkmal eines Verteilungszustands einer Zelle und den zusätzlichen Informationen und Informationen bezüglich eines malignen Lymphoms darstellen, und
Informationen bezüglich eines malignen Lymphoms auf der Basis eines Ergebnisses des maschinellen Lernens abschätzt.

10. Diagnosevorrichtung nach einem der Ansprüche 1 bis 9, wobei
die Zelle eine Gefäßendothelzelle, eine Mesenchymalzelle, einschließlich eine Fibroblastenzelle, eine Blutzelle, die von einer Lymphoidzelle verschieden ist, einschließlich ein Histiocyt, eine Epithelzelle oder eine Nervenzelle umfasst.

11. Informationserfassungsverfahren, das durch eine Informationsverarbeitungsvorrichtung durchgeführt wird, wobei die Verarbeitung durch eine Diagnosevorrichtung nach einem der Ansprüche 1 bis 10 durchgeführt wird, wobei das Verfahren umfasst:
Extrahieren eines einzelnen Zellkerns, einer einzelnen Zelle und eines Verteilungszustands einer Zelle aus einem pathologischen Bild, in dem eine Mehrzahl von Zellen, einschließlich eine Lymphoidzelle, fotografiert ist; und
Digitalisieren eines Merkmals eines einzelnen Zellkerns, eines Merkmals einer einzelnen Zelle und eines Merkmals eines Verteilungszustands einer Zelle auf der Basis des extrahierten einzelnen Zellkerns, der extrahierten einzelnen Zelle und des extrahierten Verteilungszustands einer Zelle.

12. Programm, das bewirkt, dass ein Computer als Diagnosevorrichtung nach einem der Ansprüche 1 bis 10 dient.

## Revendications

1. Dispositif de diagnostic comprenant :
un extracteur qui extrait un noyau de cellule individuelle, une cellule individuelle et un état de distribution de cellule d'une image pathologique dans laquelle est photographiée une pluralité de cellules incluant une cellule lymphoïde ; et
un numériseur qui numérise une caractéristique d'un noyau de cellule individuelle, une caractéristique d'une cellule individuelle et une caractéristique d'un état de distribution de cellule sur la base du noyau de cellule individuelle, de la cellule individuelle et de l'état de distribution de cellule extraits par l'extracteur,
comprenant en outre :
un estimateur qui estime des informations sur un lymphome malin sur la base d'une caractéristique d'un noyau de cellule individuelle, d'une caractéristique d'une cellule individuelle et d'une caractéristique d'un état de distribution la cellule, numérisés par le numériseur, dans lequel l'estimateur
effectue un apprentissage automatique sur la base des données d'échantillon représentant une relation entre une caractéristique d'un noyau de cellule individuelle, une caractéristique d'une cellule individuelle et une caractéristique d'un état de distribution de cellule, et des informations sur le lymphome malin, et
estime des informations sur le lymphome malin d'une caractéristique d'un noyau de cellule individuelle, d'une caractéristique d'une cellule individuelle et d'une caractéristique d'un état de distribution de la cellule, numérisés par le numériseur, sur la base d'un résultat de l'apprentissage automatique, et dans lequel
l'estimateur estime des informations sur le lymphome malin sur la base non seulement d'un noyau de cellule individuelle, d'une caractéristique d'une cellule individuelle et d'une caractéristique d'un état de distribution de cellule, numérisés par le numériseur, mais également au moins d'informations supplémentaires parmi des informations génomiques, des informations d'expression génique, des informations protéiques et des informations cliniques corrélées avec le lymphome malin.

2. Dispositif de diagnostic selon la revendication 1, dans lequel
l'extracteur
lit une image pathologique obtenue en prenant une plage dans laquelle une caractéristique d'un état de distribution de cellule est reconnaissable, à une résolution à laquelle les caractéristiques d'un noyau de cellule individuelle et d'une cellule individuelle sont reconnaissables, et
extrait un noyau de cellule individuelle, une cellule individuelle et un état de distribution de cellule de l'image pathologique lue.

3. Dispositif de diagnostic selon la revendication 2, dans lequel
l'extracteur
effectue un apprentissage automatique sur la base des données d'échantillon représentant une relation entre une image d'échantillon en tant qu'échantillon de l'image pathologique, et un noyau de cellule individuelle, une cellule individuelle et un état de distribution de cellule extraits manuellement de l'image d'échantillon, et
extrait un noyau de cellule individuelle, une cellule individuelle et un état de distribution de cellule de l'image pathologique, sur la base d'un résultat de l'apprentissage automatique.

4. Dispositif de diagnostic selon la revendication 1, dans lequel
l'extracteur extrait non seulement une cellule individuelle, un noyau de cellule individuelle et un état de distribution de cellule d'une première image ayant une résolution à laquelle un noyau de cellule individuelle et une cellule individuelle prélevés sur un organisme vivant sont reconnaissables, mais extrait également un état de distribution de cellule d'une seconde image contenant une plage dans laquelle une caractéristique d'un état de distribution de cellule prélevée sur l'organisme vivant est reconnaissable.

5. Dispositif de diagnostic selon la revendication 4, dans lequel
l'extracteur
effectue non seulement un apprentissage automatique sur la base des données d'échantillon représentant une relation entre une première image d'échantillon correspondant à la première image, et une cellule et un noyau de cellule extraits manuellement de la première image d'échantillon, mais effectue également un apprentissage automatique sur la base de données d'échantillon représentant une relation entre une seconde image d'échantillon correspondant à la seconde image, et un état de distribution de cellule extrait manuellement de la seconde image d'échantillon, et
extrait non seulement une cellule individuelle et un noyau de cellule individuelle de la première image, mais extrait également un état de distribution de cellule de la seconde image, sur la base d'un résultat de l'apprentissage automatique.

6. Dispositif de diagnostic selon l'une quelconque des revendications 1 à 4, dans lequel
le numériseur numérise une caractéristique d'un noyau de cellule individuelle, une caractéristique d'une cellule individuelle et une caractéristique d'un état de distribution de cellule corrélés avec des informations sur le lymphome malin, sur la base du noyau de cellule individuelle, de la cellule individuelle et de l'état de distribution de cellule extraits par l'extracteur.

7. Dispositif de diagnostic selon la revendication 6, dans lequel
le numériseur
effectue un apprentissage automatique sur la base des données d'échantillon représentant une relation entre une caractéristique d'un noyau de cellule individuelle, une caractéristique d'une cellule individuelle et une caractéristique d'un état de distribution de cellule, et des informations sur le lymphome malin, et
restreint une caractéristique d'un noyau de cellule individuelle, une caractéristique d'une cellule individuelle et une caractéristique d'un état de distribution de cellule, à une caractéristique à numériser, sur la base d'un résultat de l'apprentissage automatique.

8. Dispositif de diagnostic selon l'une quelconque des revendications 1 à 7, dans lequel
les informations sur le lymphome malin comportent des informations représentant la présence/l'absence, une classe ou un stade d'évolution du lymphome malin, et/ou des informations génomiques, et/ou des informations sur l'expression génique, et/ou des informations protéiques et/ou des informations cliniques corrélées avec le lymphome malin.

9. Dispositif de diagnostic selon l'une quelconque des revendications 1 à 8, dans lequel
l'estimateur
effectue un apprentissage automatique sur la base des données d'échantillon représentant une relation entre une caractéristique d'un noyau de cellule individuelle, une caractéristique d'une cellule individuelle et une caractéristique d'un état de distribution de cellule et les informations supplémentaires, et les informations sur le lymphome malin, et
estime les informations sur le lymphome malin sur la base d'un résultat de l'apprentissage automatique.

10. Dispositif de diagnostic selon l'une quelconque des revendications 1 à 9, dans lequel
la cellule comporte une cellule endothéliale vasculaire, une cellule mésenchymateuse comportant un fibroblaste, une cellule sanguine autre qu'une cellule lymphoïde, y compris un histiocyte, une cellule épithéliale ou une cellule neurale.

11. Procédé d'acquisition d'informations exécuté par un dispositif de traitement de l'information, dans lequel le traitement est exécuté par un dispositif de diagnostic selon l'une quelconque des revendications 1 à 10, le procédé comprenant :
l'extraction d'un noyau de cellule individuelle, d'une cellule individuelle et d'un état de distribution de cellule d'une image pathologique dans laquelle une pluralité de cellules incluant une cellule lymphoïde est photographiée ; et
la numérisation d'une caractéristique d'un noyau de cellule individuelle, d'une caractéristique d'une cellule individuelle et
d'une caractéristique d'un état de distribution de cellule sur la base du noyau de cellule individuelle extrait, de la cellule individuelle extraite et de l'état de distribution de cellule extrait.

12. Programme amenant un ordinateur à servir de dispositif de diagnostic selon l'une quelconque des revendications 1 à 10.
